# EUROPEAN PATENT SPECIFICATION

(11) **EP 4 226 958 B1**
(45) Date of publication and mention of the grant of the patent: **29.04.2026**
(21) Application number: 22842389.3
(22) Date of filing: 11.07.2022
(51) Int. Cl.: A61M 11/00, A61M 19/00, A61M 35/00, A61F 7/00, B05B 12/12, A45D 34/04, A45D 34/00, A61M 11/06, B05B 7/24

(54) **SYSTEM FOR MIXING AND SPRAYING COOLANT AND COMPOSITION**
SYSTEM ZUM MISCHEN UND VERSPRÜHEN VON KÜHLMITTEL UND ZUSAMMENSETZUNG
SYSTÈME PERMETTANT DE MÉLANGER ET DE PULVÉRISER UN FLUIDE DE REFROIDISSEMENT ET UNE COMPOSITION

(30) Priority: 12.07.2021 KR 20210091238; 01.07.2022 KR 20220081063
(43) Date of publication of application: 16.08.2023
(60) Divisional application: 26165594.8
(73) Proprietor: Recensmedical, Inc., Gyeonggi-do 18468 (KR)
(72) Inventor: RO, Kyongkwan, Hwaseong-si, Gyeonggi-do 18466 (KR); PARK, Boo Seong, Hwaseong-si, Gyeonggi-do 18466 (KR); LEE, Chulho, Yongin-si, Gyeonggi-do 17117 (KR)
(74) Representative: Walaski, Jan Filip
(86) International application number: PCT/KR2022/010017
(87) International publication number: WO 2023/287127

(56) References cited:
- WO-A1-2019/016105
- CN-A- 105 435 982
- JP-A- H08 215 614
- JP-A- H08 215 614
- KR-A- 20170 142 194
- KR-A- 20190 090 157
- KR-U- 20200 002 813
- KR-U- 20200 002 813

## Description

### Technical Field

The present disclosure relates to a system for mixing and spraying a refrigerant and a composition and, particularly, to a structure or system designed to be capable of mixing and spraying a composition and a refrigerant to improve the penetration of the composition sprayed onto the skin.

### Background Art

Generally, in the fields of beauty and medical devices, a method of effectively delivering a composition including an active ingredient to a target by spraying the same to the target has been considered a very important task, and research thereon is being actively conducted.

Meanwhile, in the case of considering the temperature of a composition when effectively delivering the composition to a target, there is a lack of research on the technology of cooling and delivering the composition particularly to improve the penetration of the composition.

This is because it is difficult to control a temperature of a composition because compressed air is mainly used to spray the composition, and furthermore, it is difficult to add a separate temperature control means in addition to a compressor when considering the size of a spraying apparatus.

As described above, the technology of spraying a composition of a low temperature is difficult to be implemented with existing technologies. In the present specification, the technology of spraying a composition having a lowered temperature to a target, in particular, the technology of spraying a composition lowered to a specific temperature by using a refrigerant is described, and a method of implementing the technology that was difficult to be implemented in the past is proposed.
KR 2020-0002813U presents a treatment apparatus for extremely low temperature including a bracket having multi-shapes.
JPH 08215614 A presents a droplet atomizer which promotes atomization of droplets by the action of an ejector.

### Disclosure

### Technical Problem

An objective to be achieved by the present specification is to provide an apparatus for mixing and spraying a composition containing an effective ingredient and a refrigerant, and a method using the same.

An objective to be achieved by the present specification is to provide a multi-function module that is coupled to a refrigerant supply device so as to spray a composition together with a refrigerant.

An objective to be achieved by the present specification is to provide is to provide an apparatus having structure in which a composition is moved at negative pressure according to the spraying of a refrigerant.

Objectives to be achieved by the present specification are not limited to the above-mentioned objectives, and objectives not mentioned will be clearly understood by those skilled in the art to which the present disclosure belongs from the present specification and the accompanying drawings.

### Technical Solution

According to one embodiment of the present specification, there is provided a multi-function module configured to be coupled to a refrigerant supply device, mixing a composition with a refrigerant supplied from the refrigerant supply device and spraying the mixture to an outside, the multi-function module including: a mixing unit providing a mixing space for the refrigerant and the composition; a combining unit comprising a combining means to combine with the refrigerant supply device; a spraying unit disposed between the mixing space and the combining means and configured to spray the refrigerant supplied from the refrigerant supply device into the mixing space; and an inlet unit providing a flow path of the composition to the mixing space, wherein the spraying unit comprises an inlet hole having a first diameter and an outlet hole having a second diameter smaller than the first diameter, the refrigerant sprayed through the outlet hole forms a negative pressure at a second end of the inlet unit such that the composition moves into the flow path through a first end of the inlet unit and flows into the mixing space through the second end of the inlet unit, the refrigerant sprayed through the spraying unit has a unique spraying shape formed along a central axis of the spraying unit, wherein the spraying shape has a spraying area perpendicular to the central axis of the spraying unit and having an area determined according to a distance between the spraying area and the outlet hole, wherein the spraying area is separated into a first section and a second section, wherein in the first section, the area of the spraying area increases as the distance between the spraying area and the outlet hole increases, and in the second section, the area of the spraying area corresponds to a width of the mixing space, and the second end of the inlet unit is formed at a position allowing the composition to flow into the second section.

According to another embodiment of the present specification, there is provided a system for mixing and spraying, the system including: a container storing a composition; a refrigerant storage unit storing a refrigerant; the mixing unit providing the mixing space for the composition and the refrigerant; the inlet unit providing the flow path for the composition to move between the container and the mixing space, wherein the first end of the inlet unit is connected to the container and the second end of the inlet unit is connected to the mixing space; and the spraying unit connected to the mixing space so as to receive the refrigerant from the refrigerant storage unit and spray the refrigerant into the mixing space, wherein the spraying unit comprises the inlet hole into which the refrigerant flows and the outlet hole through which the refrigerant is sprayed, wherein the refrigerant sprayed through the spraying unit has the unique spraying shape formed along the central axis of the spraying unit, wherein the spraying shape has the spraying area perpendicular to the central axis of the spraying unit and having an area determined according to the distance between the spraying area and the outlet hole, wherein the spraying area is separated into the first section and the second section, wherein in the first section, the area of the spraying area increases as the distance between the spraying area and the outlet hole increases, and in the second section, the area of the spraying area corresponds to the width of the mixing space, the second end of the inlet unit is formed at the position allowing the composition to flow into the second section, and a negative pressure is formed at the second end of the inlet unit according to the spraying shape of the refrigerant such that the composition flows from the container into the mixing space through the inlet unit.

The solution of this specification is not limited to the above-mentioned solution, and solutions not mentioned will be able to be clearly understood by those skilled in the art from this specification and the accompanying drawings.

### Advantageous Effects

According to the embodiment of the present specification, a cooled composition can be sprayed onto the skin so as to improve the penetrating effect of the composition to the skin.

According to the embodiment of the present specification, it is possible to provide a relatively small spraying apparatus by controlling the spraying of a composition without a separate fluid pressurizing device such as a pump.

According to the embodiment of the present specification, it is possible to provide a structure in which a refrigerant and a composition are mixed with each other by using the flow of a refrigerant.

Effects according to the present specification are not limited to the above-described effects, and effects not mentioned may be clearly understood by those skilled in the art from the present specification and the accompanying drawings.

### Description of Drawings

FIG. 1 is a view illustrating a mixing and spraying system according to one embodiment of the present specification.
FIG. 2 is a view illustrating a refrigerant supply device according to the embodiment of the present specification.
FIG. 3 is a view illustrating the configuration of the refrigerant supply device according to the embodiment of the present specification.
FIG. 4 is a view illustrating a process in which a portion of the refrigerant supply device is replaced with a multi-function module according to the embodiment of the present specification.
FIG. 5 is a view illustrating the multi-function module according to the embodiment of the present specification.
FIG. 6 is a view illustrating an adapter according to the embodiment of the present specification.
FIG. 7 is a view illustrating a composition container according to the embodiment of the present specification.
FIG. 8 is a view illustrating a mixing module according to the invention of the present specification.
FIG. 9 is a flowchart illustrating a method of mixing and spraying a composition and a refrigerant according to the embodiment of the present specification.
FIG. 10 is a view illustrating a process in which a composition supply module is prepared according to the embodiment of the present specification.
FIG. 11 is an exploded view of the refrigerant supply device and the mixing module according to the invention of the present specification.
FIG. 12 is a view illustrating a process in which the multi-function module is coupled to the refrigerant supply device according to the embodiment of the present specification.
FIG. 13 is a view illustrating the sections of some components of the mixing and spraying system according to the embodiment of the present specification.
FIG. 14 is a view illustrating a spraying unit and a spraying shape of a refrigerant according to the embodiment of the present specification.
FIG. 15 is a view illustrating a process in which a refrigerant and a composition are mixed with each other according to the embodiment of the present specification.
FIG. 16 is a view illustrating a case in which a refrigerant and a composition are not mixed with each other according to the embodiment of the present specification.
FIG. 17 is a view illustrating a relation between the position of the mixing module of the multi-function module and the position of a sensor unit of the refrigerant supply device according to the embodiment of the present specification.

### Best Mode

According to one embodiment of the present specification, there may be provided a multi-function module configured to be coupled to a refrigerant supply device, mixing a composition with a refrigerant supplied from the refrigerant supply device and spraying the mixture to an outside, the multi-function module including: a mixing unit providing a mixing space for the refrigerant and the composition; a combining unit comprising a combining means to combine with the refrigerant supply device; a spraying unit disposed between the mixing space and the combining means and configured to spray the refrigerant supplied from the refrigerant supply device into the mixing space; and an inlet unit providing a flow path of the composition to the mixing space, wherein the spraying unit comprises an inlet hole having a first diameter and an outlet hole having a second diameter smaller than the first diameter, the refrigerant sprayed through the outlet hole forms a negative pressure at a second end of the inlet unit such that the composition moves into the flow path through a first end of the inlet unit and flows into the mixing space through the second end of the inlet unit, the refrigerant sprayed through the spraying unit has a unique spraying shape formed along a central axis of the spraying unit, wherein the spraying shape has a spraying area perpendicular to the central axis of the spraying unit and having an area determined according to a distance between the spraying area and the outlet hole, wherein the spraying area is separated into a first section and a second section, wherein in the first section, the area of the spraying area increases as the distance between the spraying area and the outlet hole increases, and in the second section, the area of the spraying area corresponds to a width of the mixing space, and the second end of the inlet unit is formed at a position allowing the composition to flow into the second section.

In the second section, the area of a first spraying area corresponding to a first point corresponds to the width of the mixing space at the first point.

The length of the first section is determined by a critical length when the area of the spraying area corresponds to the width of the mixing space.

The critical length is set based on at least the second diameter of the outlet hole, the exit angle of the outlet hole, and the pressure of the refrigerant supplied.

When the second diameter is 0.15mm, the exit angle is 0°, and the pressure of the refrigerant is 60 bar, a distance from the outlet hole to the second end of the inlet unit is 9.omm or more.

The multi-function module further includes an adapter into which the inlet unit is built, the adapter including a body in which the container accommodating the composition is mounted.

The mixing unit includes a slide part, and the adapter includes a slide groove which is slidably coupled to the slide part of the mixing unit.

The adapter includes an adapter combining unit having the slide groove, wherein at least a portion of the inlet unit is located inside the adapter combining unit.

The adapter includes an auxiliary guide unit, wherein the auxiliary guide unit protrudes compared to the mixing unit.

The multi-function module includes a support member which extends from the mixing unit and supports the container containing the composition.

The combining means is a male screw or a female screw such that the combining unit and the refrigerant supply device are coupled to each other through screw coupling.

The multi-function module further includes a supporting unit disposed between the inlet hole of the spraying unit and the refrigerant supply device, wherein the supporting unit includes a hollow for the refrigerant to move, wherein the diameter of the hollow is equal to or greater than the first diameter.

According to another embodiment of the present specification, there may be provided a system for mixing and spraying, the system including: a container storing a composition; a refrigerant storage unit storing a refrigerant; a mixing unit providing a mixing space for the composition and the refrigerant; an inlet unit providing a flow path for the composition to move between the container and the mixing space, wherein a first end of the inlet unit is connected to the container and a second end of the inlet unit is connected to the mixing space; and a spraying unit connected to the mixing space so as to receive the refrigerant from the refrigerant storage unit and spray the refrigerant into the mixing space, wherein the spraying unit comprises an inlet hole into which the refrigerant flows and an outlet hole through which the refrigerant is sprayed, wherein the refrigerant sprayed through the spraying unit has a unique spraying shape formed along a central axis of the spraying unit, wherein the spraying shape has a spraying area perpendicular to the central axis of the spraying unit and having an area determined according to a distance between the spraying area and the outlet hole, wherein the spraying area is separated into a first section and a second section, wherein in the first section, the area of the spraying area increases as the distance between the spraying area and the outlet hole increases, and in the second section, the area of the spraying area corresponds to a width of the mixing space, the second end of the inlet unit is formed at a position allowing the composition to flow into the second section, and a negative pressure is formed at the second end of the inlet unit according to the spraying shape of the refrigerant such that the composition flows from the container into the mixing space through the inlet unit.

### Mode for Invention

The above-described objectives, features, and advantages of the present application will become more apparent through the following detailed description in relation with the accompanying drawings. However, the present application may variously be changed and may have various embodiments. Hereinafter, specific embodiments will be illustrated in the drawings and described in detail.

In the drawings, the thicknesses of layers and regions are exaggerated for clarity, and in addition, indicating that an element or layer is located "on" or "on" another component or layer may include all cases in which the element or layer is located directly on another element or layer and still another element or layer is located therebetween. Throughout the specification, like reference numerals refer to like elements in principle. In addition, components having the same function within the scope of the same idea shown in the drawings of each embodiment will be described using the same reference numerals, and overlapping descriptions thereof will be omitted.

When it is determined that a detailed description of a known function or configuration related to the present application may unnecessarily obscure the gist of the present application, the detailed description thereof will be omitted. In addition, ordinal numbers (for example, first and second, etc.) used in the description process of the present specification are only identifiers for distinguishing one component from other components.

In addition, terms "module" and "part" for components used in the following embodiments are given or mixed in consideration of only the ease of writing the specification, and do not have meanings or roles distinct from each other by themselves.

In the following embodiments, a singular expression includes a plural expression unless the context clearly dictates otherwise.

In the following embodiments, terms such as "include" or "have" mean that there are features or components described in the specification, and do not preclude the possibility that one or more other features or components may be added.

In the drawings, the size of each component may be exaggerated or reduced for convenience of description. For example, the size and thickness of each component shown in the drawings are arbitrarily indicated for convenience of description, and the present disclosure is not necessarily limited thereto.

In cases in which a certain embodiment may be realized differently, a specific process sequence may be different from the described sequence. For example, two processes described in succession may be performed substantially simultaneously, or may be performed in an order opposite to the described order.

In the following embodiments, when it is said that a film, a region, and a component are connected to each other, it includes not only a case in which the film, the region, and the component are directly connected to each other, but also a case in which other film, region, and component are placed between the film, the region, and the component such that the film, the region, and the component are indirectly connected to each other.

For example, in the present specification, when it is said that a membrane, a region, and a component are electrically connected to each other, it includes not only a case in which the film, region, and component is directly electrically connected to each other, but also other membrane, region, and component are placed between the film, region, and component such that the film, region, and component are indirectly electrically connected to each other.

In the following embodiments, meaning that the membrane, region, and component, etc. are fluidly connected to each other may be interpreted as meaning that each of the membrane, region, and component, etc. forms at least a part of a flow path through which a fluid flows.

For example, in this specification, that a component A is fluidly connected to a component B means that a fluid passing through a flow path formed in the component A can reach a flow path formed in the component B or vice versa. Specifically, when the component A and the component B are coupled to each other and the flow path formed in the component A and the flow path formed in the component B are directly connected to each other, the component A and the component B may be considered to be fluidly connected to each other. Alternatively, when the component A and the component B are connected to each other through a component C, such as a conduit and the flow path formed in the component A and the flow path formed in the component B are indirectly connected to each other through a flow path formed in the component C, the component A and the component B may be considered to be fluidly connected to each other. In this case, it may be interpreted that the component C fluidly connects the component A with the component B. In addition, the component A and the component B may be fluidly connected to each other through a plurality of components.

The present specification relates to an apparatus or system for spraying a composition together with a refrigerant, and to a method of using the same. Specifically, according to the one embodiment of the present specification, an apparatus or system in which in inducing cosmetic and medical effects by spraying a composition on a target, in order to improve the penetration of the composition into the target, the composition is sprayed while the target is cooled to an appropriate temperature, or a cooled composition is sprayed, and a method of using the same may be provided.

In the present specification, a target may refer to a body part in which a cosmetic effect or a medical effect is intended to be generated through a procedure or treatment. For example, the target may mean the skin. Hereinafter, for convenience of description, a case in which the target is the skin is mainly described, but the technical idea of the present specification is not limited thereto.

In the present specification, a composition has a concept encompassing not only pharmaceutical compositions used for medical treatment purposes but also cosmetic compositions used for cosmetic purposes, and may mean substances including active ingredients that induce or generate medical and cosmetic effects.

In the present specification, as a refrigerant, a material capable of applying cooling energy to a target region, such as carbon dioxide, liquid nitrogen, nitrogen dioxide (NO2), nitrogen monoxide (NO), nitrous oxide (N2O), an HFC-based material, methane, PFC, SF6, cooling water, and cooling gas, etc. may be used.

Meanwhile, the degree of penetration of a composition into the skin may be affected by the temperature of the skin. Specifically, when the temperature of the skin is lowered to a certain level, the cells of the skin contract and the gap between the cells increases so that a composition penetrates through the gap between the cells, resulting in improved penetration of the composition.

Hereinafter, a system for mixing and spraying a refrigerant and a composition to improve the penetration of the composition into the skin and a method of using the same will be described.

### [A mixing and spraying system]

According to the one embodiment of the present specification, a system for mixing and spraying a composition and a refrigerant may be provided.

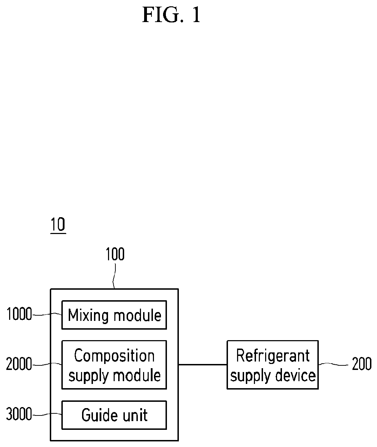

FIG. 1 is a view illustrating the mixing and spraying system 10 according to the invention of the present specification. Referring to FIG. 1, the mixing and spraying system 10 may include a multi-function module 100 including a mixing module 1000, a composition supply module 2000, and the guide unit 3000, and a refrigerant supply device 200.

First, the refrigerant supply device 200 may refer to a device that provides a refrigerant. Specifically, the refrigerant supply device 200 may supply a refrigerant to the multi-function module 100.

The refrigerant supply device 200 may store a refrigerant inside or may receive a refrigerant from a separate refrigerant storage means. For example, the refrigerant supply device 200 may be combined with a cartridge in which a refrigerant described later is stored, and may obtain the refrigerant from the combined cartridge. For another example, the refrigerant supply device 200 may receive a refrigerant from an external refrigerant reservoir through a hose.

The refrigerant supply device 200 may determine the characteristics of a supplied refrigerant. For example, the refrigerant supply device 200 may control the supply amount, supply time period, pressure and temperature of a refrigerant.

The mixing module 1000 of the multi-function module 100 may spray a refrigerant provided from the refrigerant supply device 200 together with a composition. The mixing module 1000 may include a combining unit combined with the refrigerant supply device 200, a spraying unit for spraying a supplied refrigerant, and a mixing unit providing a space in which a refrigerant and a composition are mixed.

The composition supply module 2000 of the multi-function module 100 may supply a composition. The composition supply module 2000 may include a composition container in which the composition is accommodated, and an adapter fluidly connecting the composition container and the mixing module 1000 to each other. The composition may move from the composition container through the adapter to the mixing module 1000.

The guide unit 3000 of the multi-function module 100 may contribute to fixing the spraying distance of a refrigerant and a composition to the skin. For example, when using the mixing and spraying system 10, in a state in which a certain distance between the multi-function module 100 and the skin is maintained while an end of the guide unit 3000 is in contact with the skin, a refrigerant and a composition may be sprayed.

In the multi-function module 100, some components may be omitted. For example, the multi-function module 100 includes the mixing module 1000 and the composition supply module 2000, and may separately have the guide unit 3000. For another example, the multi-function module 100 includes the mixing module 1000 and the adapter, and may separately have the composition container. The structure and function of each component of the multi-function module 100 will be described in detail later.

### [The refrigerant supply device]

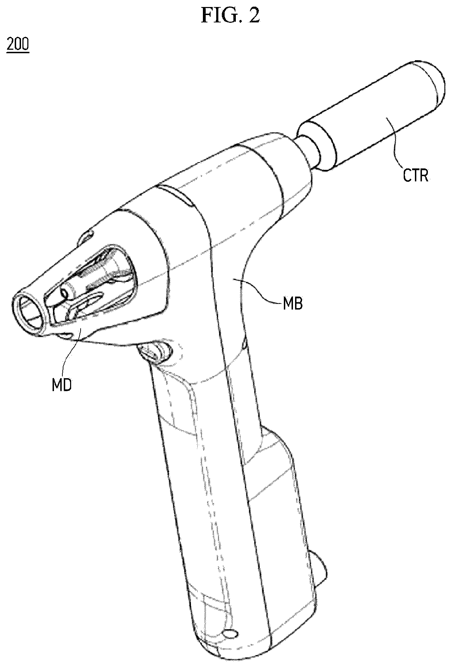

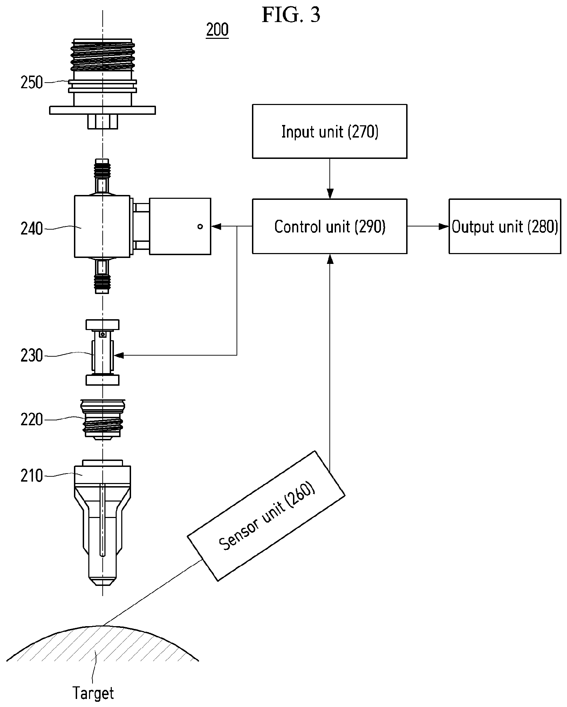

Hereinafter, the refrigerant supply device 200 will be described with reference to FIGS. 2 and 3.

FIG. 2 is a view illustrating the refrigerant supply device 200 according to the embodiment of the present specification, and FIG. 3 is a view illustrating the configuration of the refrigerant supply device 200 according to the embodiment of the present specification.

Referring to FIGS. 2 and 3, the refrigerant supply device 200 is apparently divided into a main body MB, a distance maintaining unit MD, a nozzle 210, and the cartridge CTR, wherein the main body MB may include a module combining unit 220, a temperature control unit 230, a flow rate control unit 240, a cartridge combining unit 250, a sensor unit 260, an input unit 270, an output unit 280, and a control unit 290.

The cartridge CTR can store a refrigerant. The cartridge CTR may include a combining means for combining with the cartridge combining unit 250 of the main body MB.

The cartridge CTR can store a refrigerant under a certain pressure. For example, pressure in the cartridge CTR may be determined between about 35 and 100bar based on 0 to 40°. The pressure in the cartridge CTR may preferably be determined between about 50bar and 72bar based on 15 to 30°. The pressure in the cartridge CTR may affect the spraying shape of a refrigerant as described later.

When a refrigerant is sprayed to a target through the refrigerant supply device 200, the distance maintaining part MD is intended to maintain a distance between the target and the refrigerant supply device 200, and may be understood as performing a function similar to the function of the guide unit 3000 of the multi-function module 100.

The nozzle 210 may spray a refrigerant. For example, the nozzle 210 has a flow path formed by extending from a first end thereof to a second end thereof, and includes a relatively narrow part of the flow path. A fluid passing through the nozzle expands as the fluid has decreased pressure while passing through the narrow part, and, as a result, may be sprayed at high speed. As a refrigerant passes through the nozzle 210, the refrigerant expands adiabatically and has a very low temperature. As will be described later, the temperature of the sprayed refrigerant may be controlled to a desired temperature through the temperature control unit 230. The nozzle 210 may be attached to and detached from the refrigerant supply device 200 and as described later, may be replaced with at least a component of the multi-function module 100.

The module combining unit 220 may be connected to various types of modules. A function or effect that can be generated by using the refrigerant supply device 200 may be determined according to a module connected to the module combining unit 220. For example, when the nozzle 210 for spraying refrigerant is coupled to the module combining unit 220, the refrigerant supply device 200 may function as a refrigerant spraying device for spraying a refrigerant. For another example, when the multi-function module 100 is combined with the module combining unit 220, a mixing and spraying apparatus for mixing and spraying a refrigerant and a composition can be configured.

The module combining unit 220 may provide a flow path through which a refrigerant moves. For example, the module combining unit 220 includes an outflow hole, and a refrigerant supplied from the cartridge CTR may be provided through the outflow hole of the module combining unit 220 to the outside or to a module coupled to the module combining unit 220.

The temperature control unit 230 may control the temperature of a refrigerant. The temperature control unit 230 may increase the temperature of a refrigerant by providing thermal energy to the refrigerant, and the temperature of the refrigerant may be controlled according to the amount of the thermal energy provided by the temperature control unit 230. Specifically, the temperature control unit 230 may include a heat generating unit that generates thermal energy and a heat transfer unit that transfers the generated thermal energy to a flow path through which a refrigerant moves. The heat generating unit may include a device using a thermoelectric effect such as a Peltier's effect to generate thermal energy according to applied power.

The flow rate control unit 240 may control the movement of a refrigerant. For example, the flow rate control unit 240 includes a valve, and a refrigerant may or may not move depending on whether the valve is opened or closed, and furthermore, the degree of refrigerant movement may be determined according to the degree of the opening and closing of the valve.

The cartridge combining unit 250 may accommodate at least a portion of the cartridge CTR. With the cartridge CTR coupled to the cartridge combining unit 250, a refrigerant stored in the cartridge CTR may move to the main body MB.

The sensor unit 260 may measure the temperature of a part to which a refrigerant is sprayed. For example, the sensor unit 260 may measure the temperature of a skin surface onto which a refrigerant is sprayed and provide the measurement information to the control unit 290.

The input unit 270 may receive a user's input. For example, the input unit 270 may include at least one push button switch and may provide a push input signal to the control unit 290 according to a user pressing the switch, and the control unit 290 may control the opening and closing of the flow rate control unit 240 based on the push input signal. In addition, the input unit 270 may include at least one rotary switch and provide a rotation input signal to the control unit 290 according to a user's manipulation, and the control unit 290 may set a target cooling temperature or a target cooling time period based on the rotation input signal. Here, the target cooling temperature may refer to a temperature of a target to which a refrigerant is intended to be sprayed, for example, a temperature of a skin surface to be cooled. In addition, the target cooling time period may mean a time period for which the spraying of a refrigerant is required to be maintained or a time period required to elapse while the temperature of a skin surface reaches the target cooling temperature.

The output unit 280 may output an interface for using the refrigerant supply device 200 and various pieces of information to a user. For example, the output unit 280 may include a display, and may output an interface for setting the target cooling temperature and the target cooling time period, etc. through the display, and may output information about the real-time temperature of a skin surface measured by the sensor unit 260 or the total spraying time period of a refrigerant during the operation of the refrigerant supply device 200.

The control unit 290 may control the components of the refrigerant supply device 200. For example, the control unit 290 may control the temperature of a refrigerant by controlling the temperature control unit 230, may control the flow of a refrigerant by controlling the flow rate control unit 240, and may output specific information to a user through the output unit 280.

Referring to FIG. 3, the refrigerant supply device 200 may operate as follows.

The control unit 290 may first set the target cooling temperature and the target cooling time period. The control unit 290 may provide an interface for inducing the setting of the target cooling temperature and the target cooling time period to a user through the output unit 280, may receive a set input signal according to a user's manipulation through the input unit 270, and may set the target cooling temperature and the target cooling time period based on the received set input signal.

After that, the control unit 290 may output a message indicating that operation preparation is completed to a user through the output unit 280, and may receive the input signal of switch on according to a user's manipulation through the input unit 270, and may spray refrigerant based on the received switch-on input signal.

The control unit 290 may obtain a temperature value of a target, to which a refrigerant is sprayed, measured through the sensor unit 260 while a refrigerant is sprayed and may compare the obtained temperature value with a set target cooling temperature so that the temperature control unit 230 can be controlled. In this case, the control unit 290 may increase thermal energy applied to a refrigerant through the temperature control unit 230 when the obtained temperature value is lower than the target cooling temperature, but may decrease thermal energy applied to a refrigerant through the temperature control unit 230 when the obtained temperature value is higher than the target cooling temperature.

The refrigerant supply device 200 is not limited to the above-described embodiment, and any device or structure that performs a function of supplying a refrigerant may be regarded as the refrigerant supply device 200 described in the present specification. For example, the refrigerant supply device 200 may not control the temperature of a refrigerant, and in this case, the temperature control unit 230 and the sensor unit 260 may be omitted.

### [The multi-function module]

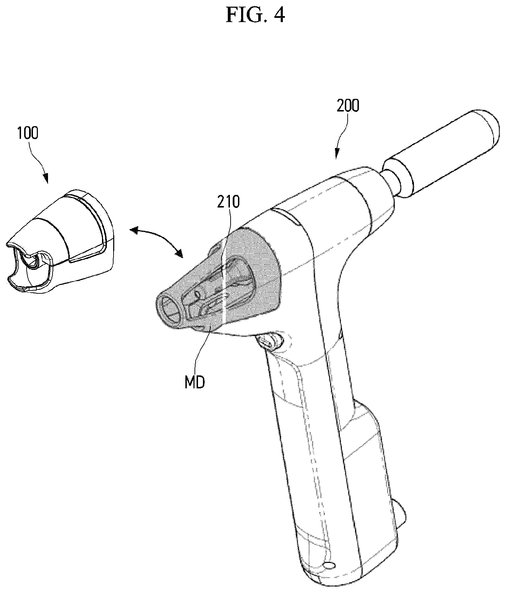

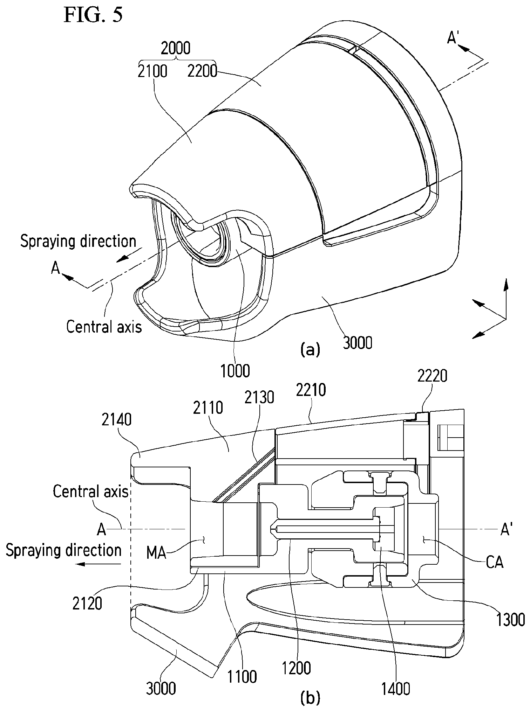

Hereinafter, an aspect in which the multi-function module 100 is coupled to the above-described refrigerant supply device 200 and the configuration of the multi-function module 100 will be described with reference to FIGS. 4 and 5.

FIG. 4 is a view illustrating a process in which a portion of the refrigerant supply device 200 is replaced with the multi-function module 100 according to the embodiment of the present specification.

The multi-function module 100 may be provided by being coupled to the refrigerant supply device 200. Specifically, at least a part of the multi-function module 100 may be replaced with a part of the refrigerant supply device 200. For example, referring to FIG. 4, the nozzle 210 and the distance maintaining part MD of the refrigerant supply device 200 may be replaced with the multi-function module 100. For another example, the nozzle 210 of the refrigerant supply device 200 may be replaced with the mixing module 1000 and the composition supply module 2000 of the multi-function module 100.

The mixing and spraying system 10 described in this specification is not necessarily required to be formed in a form in which the multi-function module 100 and the refrigerant supply device 200 are combined with each other, and may be formed as an integrated unit.

FIG. 5 is a view illustrating the multi-function module 100 according to the invention of the present specification. Referring to FIG. 5, the multi-function module 10 includes the mixing module 1000, the composition supply module 2000, and the guide unit 3000. Here, the composition supply module 2000 is divided into the adapter 2100 and the composition container 2200.

Each component of the multi-function module 100 may be physically separated from each other. For example, the mixing module 1000, the composition supply module 2000, and the guide unit 3000 may be physically separated from each other. For another example, the adapter 2100 and the composition container 2200 may be physically separated from each other and may be assembled to constitute the composition supply module 2000.

Meanwhile, at least two components of components of the multi-function module 100 may not be physically separated from each other. For example, the mixing module 1000 and the composition supply module 2000 may be an integral module that is not physically separated. For another example, the mixing module 1000 and the adapter 2100 may be an integral module that is not physically separated. For another example, the adapter 2100 and the composition container 2200 may be an integral module.

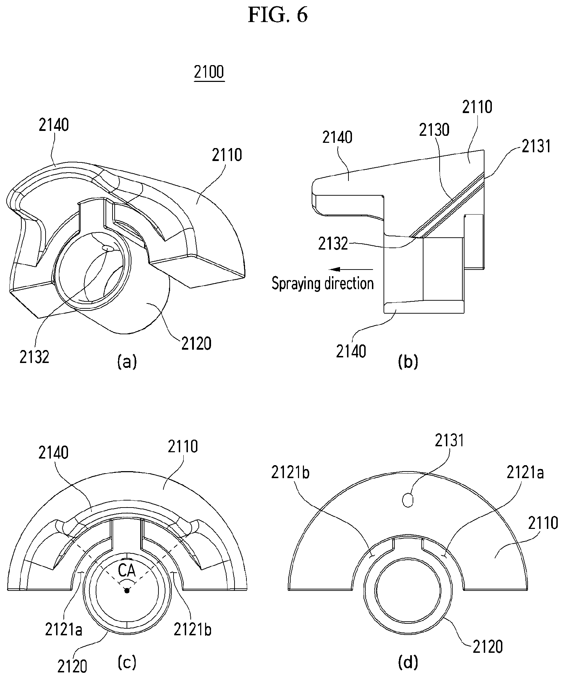

Hereinafter, each component of the multi-function module 100 will be described in detail with reference to FIGS. 6 to 8.

FIG. 6 is a view illustrating the adapter 2100 according to the embodiment of the present specification. Referring to FIG. 6, the adapter 2100 may include an adapter body 2110, an adapter combining unit 2120, an inlet unit 2130, and an auxiliary guide unit 2140.

The adapter body 2110 may be coupled to the composition container 2200. The adapter body 2110 may be coupled to the composition container 2200 by forcible fitting, screwing, sliding, or a magnet, and may include combining means required for each coupling.

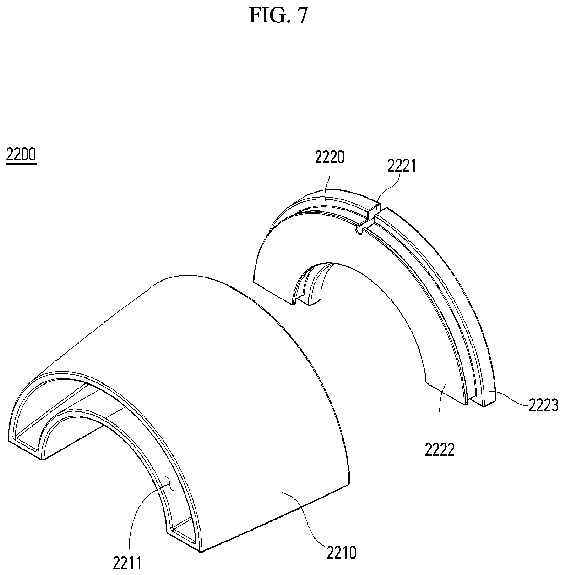

The adapter body 2110 may have a shape corresponding to the composition container 2200. For example, referring to FIGS. 6 and 7, when an opening part of the composition container 2200 has a semi-annular shape, one cross-sectional area of the adapter body 2110 may have a semi-annular shape. For another example, when the composition container 2200 is circular, at least a part of the adapter body 2110 may also be circular. A detailed process in which the adapter body 2110 and the composition container 2200 are coupled to each other will be described later.

The adapter combining unit 2120 may be coupled to the mixing module 1000. The adapter combining unit 2120 may be coupled to the mixing unit of the mixing module 1000 by sliding, forcible fitting, screwing, or a magnet, and may include combining means required for each coupling.

The adapter combining unit 2120 may include a shape corresponding to the mixing module 1000. For example, in a case in which the adapter combining unit 2120 is coupled to the mixing unit of the mixing module 1000 as described later, when the mixing unit has a cylindrical shape having a specific diameter, the adapter combining unit 2120 may include a cylindrical part having a diameter smaller than the diameter of the mixing unit.

The adapter combining unit 2120 may constitute at least a part of a mixing space MA. For example, as illustrated in FIG. 5(b), when the adapter 2100 is mounted to the mixing module 1000 and a portion of the adapter combining unit 2120 is located inside the mixing unit 1200, a space surrounded by the adapter combining unit 2120 may constitute at least a part of the mixing space MA. Here, the adapter combining unit 2120 may be formed in a half-ring shape or a curved surface shape in addition to a ring shape illustrated in FIG. 6 and may constitute a part of the mixing space MA.

As described above, the adapter combining unit 2120 constitutes at least a part of the mixing space MA, and thus a composition may directly move to the mixing space MA through the inlet unit 2130 mounted in the adapter combining unit 2120. In this case, according to the shape of the adapter combining unit 2120 or the position of the inlet unit 2130 in the adapter combining unit 2120, the introducing position and direction of a composition into the mixing space MA may be determined. In other words, the adapter combining unit 2120 constitutes at least a part of the mixing space MA, and thus in the mixing space MA, a positional relationship between the inflow path of a composition and the spraying path of a refrigerant may be more easily specified.

The adapter combining unit 2120 may be connected to the adapter body 2110. For example, the adapter combining unit 2120 may extend from the adapter body 2110 and may not be physically separated from the adapter body 2110. For another example, the adapter combining unit 2120 may be physically separated from the adapter body 2110 and assembled therewith.

The adapter 2100 may be coupled to the mixing module 1000 through the adapter combining unit 2120.

For example, the adapter 2100 includes a slide space defined between the adapter combining unit 2120 and the adapter body 2110, and at least a portion of the mixing module 1000 is inserted into the slide space so that the adapter 2100 is slidably coupled to the mixing module 1000. As illustrated in FIGS. 6(c) and 6(d), the slide space may include first and second slide grooves 2121a and 2121b formed when the adapter body 2110 surrounds the adapter combining unit 2120. Alternatively, a slide rail having a protrusion or groove in at least a portion of the adapter combining unit 2120 may be used by the sliding coupling.

For another example, the adapter combining unit 2120 may be combined with the mixing module 1000 through the aforementioned screw coupling, and for this purpose, at least a portion of the adapter combining unit 2120 may be a female screw or a male screw.

The inlet unit 2130 may have a flow path through which a composition can move. For example, the inlet unit 2130 may be formed in the form of a conduit extending from the first end 2131 of the inlet unit to the second end 2132 of the inlet unit. Specifically, the first end 2131 of the inlet unit may be fluidly connected to the composition container 2200, and the second end 2132 of the inlet unit may be fluidly connected to the mixing module 1000. The inlet unit 2130 may guide the movement of the composition to the mixing module 1000 from the composition container 2200.

The inlet unit 2130 may be located inside the adapter body 2110 and/or the adapter combining unit 2120. For example, referring to FIGS. 6(a), 6(b), and 6(d), a portion of the inlet unit 2130 may be located inside the adapter body 2110, and another portion of the inlet unit 2130 may be located inside the adapter combining unit 2120. In this case, the first end 2131 of the inlet unit is formed in the adapter body 2110 coupled to the composition container 2200 as illustrated in FIGS. 6(b) and 6(d), and the second end 2132 of the inlet unit may be formed in the adapter combining unit 2120 connected to the mixing module 1000 as illustrated in 6(a) and 6(b).

When the mixing and spraying system operates, the auxiliary guide unit 2140 may assist a user to maintain a distance between the multi-function module 100 and the user's skin. The auxiliary guide unit 2140 may be formed in a form protruding from the adapter body 2110 in one direction. For example, referring to FIGS. 5 and 6, based on a state in which the adapter 2100 is coupled to the mixing module 1000, the auxiliary guide unit 2140 may protrude from the adapter body 2110 in a spraying direction in which a refrigerant and a composition are sprayed.

The auxiliary guide unit 2140 may have a shape surrounding the central axis parallel to the spraying direction. For example, referring to FIG. 6(c), the auxiliary guide unit 2140 may have a shape surrounding the central axis by a first angle relative to the central axis. In this case, the first angle by which the auxiliary guide unit 2140 surrounds the central axis may be vary according to a degree to which the mixing and spraying system 10 seals a refrigerant and a composition while operating. As the degree of sealing increases, the first angle may have a larger value. The first angle may be set between 0 degree and 360 degrees. The first angle may be preferably set between 30 degrees and 180 degrees.

The material of the auxiliary guide unit 2140 may be different from the material of the adapter body 2110. For example, the auxiliary guide unit 2140 may be made of a material having ductility greater than the ductility of the adapter body 2110.

The protruding length of the auxiliary guide unit 2140 may correspond to the length of the guide unit 3000. For example, referring to FIG. 5(b), in a state in which the adapter 2100 is coupled to the mixing module 1000, the protruding degree of the auxiliary guide unit 2140 and the protruding degree of the guide unit 3000 may correspond to each other along the spraying direction relative to the mixing module 1000. Specifically, while the adapter 2100 is coupled to the mixing module 1000, a distance between an end of the mixing module 1000 and an end of the auxiliary guide unit 2140 may be the same as a distance between the end of the mixing module 1000 and an end of the guide unit 3000. In this case, when the auxiliary guide unit 2140 and the guide unit 3000 are in contact with a surface of the skin, the spraying direction of a refrigerant may be approximately perpendicular to the surface of the skin.

The shape of the auxiliary guide unit 2140 may correspond to the shape of the guide unit 3000. For example, referring to FIG. 5(a), when the guide unit 3000 is divided into a guide connection part coupled to the refrigerant supply device 200 and a contact part to be in contact with the skin, the auxiliary guide unit 2140 and the contact part of the guide unit 3000 surrounds the central axis of the multi-function module 100 parallel to the spraying direction, and the shape of the auxiliary guide unit 2140 and the shape of the contact part of the guide unit 3000 may be symmetrical to each other relative to the central axis.

FIG. 7 is a view illustrating the composition container 2200 according to the embodiment of the present specification. Referring to FIG. 7, the composition container 2200 may be divided into a casing 2210, a cap part 2220, and a cover (not shown).

The casing 2210 may provide a composition receiving space 2211 for receiving a composition.

The casing 2210 may be coupled to the adapter 2100. For example, a first end of the casing 2210 may be coupled to the adapter body 2110. As a method of coupling the casing 2210 to the adapter 2100, screw coupling, coupling by forcible fitting, magnetic coupling, and slide coupling, etc. may be used.

The casing 2210 may have a shape corresponding to the shape of the adapter body 2110.

The cap part 2220 may be coupled to a second end of the casing 2210 and may seal at least a portion of the composition receiving space 2211.

The cap part 2220 may include a step part 2222 for combining with the casing 2210.

The cap part 2220 may include an outside air inlet unit 2221 for introducing outside air. As will be described later, the outside air inlet unit 2221 may refer to a part through which outside air is introduced while the mixing and spraying system 10 is operating. The outside air inlet unit 2221 is not required to be formed in the cap part 2220. The outside air inlet unit 2221 may be formed in a portion fluidly connected to the composition receiving space 2211, such as the casing 2210 or the adapter body 2110.

The cap part 2220 may have a shape corresponding to the casing 2210.

Meanwhile, the composition container 2200 is not limited to including the above-described components. The composition container 2200 may be a product receiving a composition, for example, a cosmetic product such as an ampoule or a vial, or a pharmaceutical product.

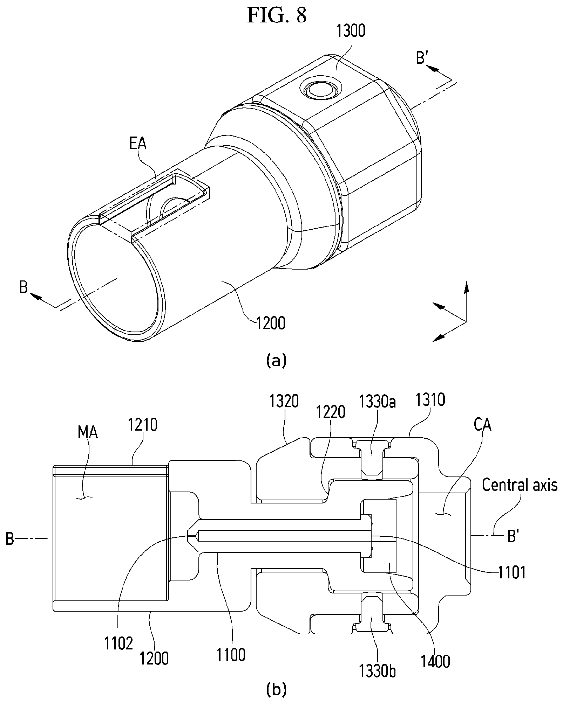

FIG. 8 is a view illustrating the mixing module 1000 according to the embodiment of the present specification. Referring to FIG. 8, the mixing module 1000 may be divided into the spraying unit 1100, the mixing unit 1200, and a combining unit 1300.

The spraying unit 1100 may spray a passing refrigerant. The spraying unit 1100 may include a part in which a width decreases. For example, the spraying unit 1100 may include an inlet hole 1101 for introducing a refrigerant and an outlet hole 1102 for spraying a refrigerant. The diameter of the inlet hole 1101 may be greater than the diameter of the outlet hole 1102. While a refrigerant passes through the spraying unit 1100, pressure thereof decreases and a moving speed thereof increases at the part in which the width decreases, so that it may be sprayed while expanding.

The spraying unit 1100 is disposed between the mixing space MA of the mixing unit 1200 and a coupling space CA of the combining unit 1300, which will be described later, and receives a refrigerant from the refrigerant supply device 200 connected to the combining unit 1300 so that the spraying unit 1100 can spray the refrigerant to the mixing space MA. The characteristics of the spraying unit 1100 and the spraying shape of a refrigerant according thereto will be described later.

The mixing unit 1200 may provide the mixing space MA for mixing a refrigerant and a composition. For example, as illustrated in FIG. 8, the mixing unit 1200 may have the mixing space MA having a cylindrical shape. The shape of the mixing space MA may vary depending on the internal shape of the mixing unit 1200 (ex. a polygonal column, and a truncated cone whose width decreases or increases toward a bottom thereof, etc.). Furthermore, as will be described later, the shape of the mixing space MA may vary depending on another component other than the mixing unit 1200 (ex. the internal space of the adapter combining unit 2120).

A refrigerant and a composition may be introduced into the mixing space MA. For example, as described above, a refrigerant sprayed through the spraying unit 1100 may flow into the mixing space MA, and a composition stored in the composition container 2200 may flow into the mixing space MA through the inlet unit 2130 of the adapter 2100. In this case, a composition may be introduced into the mixing space MA by the spraying of a refrigerant, and method and structure in which a composition is introduced will be described later.

The mixing unit 1200 may be coupled to the adapter 2100. The mixing unit 1200 may include a mounting area EA and a coupling member 1210 to be coupled to the adapter 2100. Here, the mounting area EA may refer to an area into which at least a part of the adapter 2100 is inserted or an area to which at least a part of the adapter 2100 is coupled. In addition, the coupling member 1210 may be understood as a means for combining the adapter 2100 with the mixing unit 1200.

For example, when the mixing unit 1200 and the adapter 2100 are slidably coupled to each other, as illustrated in FIG. 8, the coupling member 1210 of the mixing unit 1200 may be formed in the form of a slide plate inserted into the slide space of the adapter combining unit 2120 described above. In addition, the adapter combining unit 2120 may be inserted into the mounting area EA of the mixing unit 1200, and the coupling member 1210 of the mixing unit 1200 may be positioned between the adapter combining unit 2120 and the adapter body 2110.

For another example, when the mixing unit 1200 and the adapter 2100 are coupled to each other by screwing, the coupling member 1210 of the mixing unit 1200 may be a female screw or a male screw to be screwed to the adapter combining unit 2120.

Meanwhile, since the mixing unit 1200 includes the mounting area EA, a composition may be introduced into the mixing space MA directly from the inlet unit 2130 of the adapter 2100. In other words, when the mixing unit 1200 includes a receiving member other than the mounting area EA, a composition may pass through the inlet unit 2130 of the adapter 2100 and flow into the mixing space MA through the receiving member of the mixing unit 1200. However, in this case, in order to prevent leakage of a composition between the inlet unit 2130 and the receiving member of the mixing unit 1200, a precise design is required so that there is no gap between the inlet unit 2130 and the receiving member of the mixing unit 1200, which may increase a production cost. Accordingly, it is more preferable that the mixing unit 1200 includes the mounting area EA so that a composition directly flows into the mixing space MA through the adapter combining unit 2120.

In the above, the case in which the mounting area EA and the coupling member 1210 are included in the mixing unit 1200 has been described, but the technical spirit of the present specification is not limited thereto, and the mounting area EA and/or the coupling member 1210 may be provided in any part of the mixing module 1000. In addition, the mixing unit 1200 does not necessarily include the mounting area EA and the coupling member 1210, and when a composition is introduced into the mixing space MA through a separate flow path such as a pipe or hose, the mounting area EA and/or the coupling member 1210 may be omitted.

The mixing unit 1200 may provide an inner space in which the spraying unit 1100 is disposed. For example, referring to FIG. 8, the mixing unit 1200 may include an inner space connected to the mixing space MA, and the spraying unit 1100 may be located in the inner space of the mixing unit 1200.

The combining unit 1300 may be coupled to the refrigerant supply device 200. The combining unit 1300 may provide the coupling space CA for accommodating at least a part of the refrigerant supply device 200.

The combining unit 1300 may include a main combining part 1310 and an auxiliary combining part 1320. The main combining part 1310 provides the coupling space CA, and may be coupled to the refrigerant supply device 200 in the coupling space CA. The main combining part 1310 may include a combining means so as to be coupled to the refrigerant supply device 200. For example, the main combining part 1310 may include a male screw or female screw part so as to be screwed to the refrigerant supply device 200.

The auxiliary combining part 1320 may fix the spraying unit 1100 and the mixing unit 1200. For example, in the process of coupling the mixing module 1000 to the refrigerant supply device 200, the auxiliary combining part 1320 may press the spraying unit 1100 and the mixing unit 1200 in a direction parallel to the central axis, and may prevent the spraying unit 1100 and the mixing unit 1200 from moving after the mixing module 1000 and the refrigerant supply device 200 are coupled to each other.

Specifically, referring to FIG. 8, the mixing unit 1200 includes a holding step 1220, and when the main combining part 1310 moves in a direction parallel to the central axis while being coupled to the refrigerant supply device 200, the auxiliary combining part 1320 presses the holding step 1220 of the mixing unit 1200 while moving together with the main combining part 1310 so that the spraying unit 1100 and the mixing unit 1200 can be held while sharing a center with the refrigerant supply device 200. In addition, in this case, the main combining part 1310 rotates for screw coupling, but the mixing unit 1200 may be fixed in a non-rotating state when viewed in the central axis direction, so the composition supply module 2000 coupled to the mixing unit 1200 also may not rotate when viewed in the central axis direction. As a result, the stability of the mixing and spraying system 10 in which the multi-function module 100 and the refrigerant supply device 200 are coupled to each other improves, and furthermore, when used, the mixing and spraying system 10 is balanced, so convenience thereof can be increased.

The main combining part 1310 and the auxiliary combining part 1320 may be fixed to each other through fixing members. For example, as illustrated in FIG. 8, the main combining part 1310 and the auxiliary combining part 1320 have at least two pairs of holes corresponding to each other, and first and second fixing members 1330a and 1330b are inserted into each pair of holes so that the main combining part 1310 and the auxiliary combining part 1320 can be fixed to each other.

The supporting unit 1400 may support the spraying unit 1100. The supporting unit 1400 may be disposed between the coupling space CA of the combining unit 1300 and the spraying unit 1100. As described later, the spraying unit 1100 may be fixed to the refrigerant supply device 200 through the supporting unit 1400. The supporting unit 1400 may prevent leakage of a refrigerant in a process in which the mixing module 1000 is coupled to the refrigerant supply device 200 or a process in which the refrigerant moves from the refrigerant supply device 200 to the spraying unit 1100.

The supporting unit 1400 may provide a flow path through which a refrigerant moves. The supporting unit 1400 may include a hollow to provide a flow path through which a refrigerant moves. The size of the hollow of the supporting unit 1400 may correspond to the size of the inlet hole 1101 of the spraying unit 1100. The size of the hollow of the supporting unit 1400 may correspond to the size of the outflow hole of the module combining unit 220 of the refrigerant supply device 200. The size of the hollow of the supporting unit 1400 may correspond to the size of the inlet hole 1101 of the spraying unit 1100 at a first end of the supporting unit 1400 connected to the spraying unit 1100, and may correspond to the size of the outflow hole of the module combining unit 220 at a second end of the supporting unit 1400 connected to the module combining unit 220. In other words, depending on the size of the inlet hole 1101 of the spraying unit 1100 and the size of the outflow hole of the module combining unit 220, the sizes of the hollow of the supporting unit 1400 at the opposite ends of the supporting unit 1400 may be different or the same.

### [A method of using the mixing and spraying system]

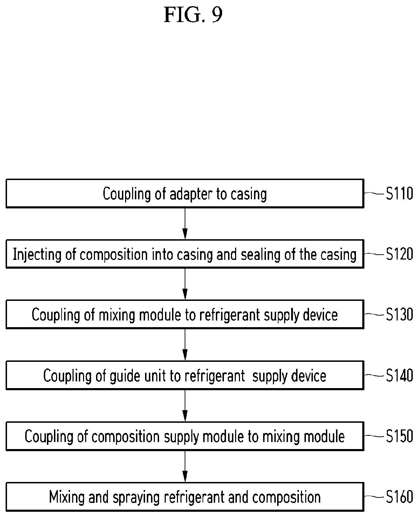

Hereinafter, a method of using the mixing and spraying system 10 will be described with reference to FIGS. 9 to 12.

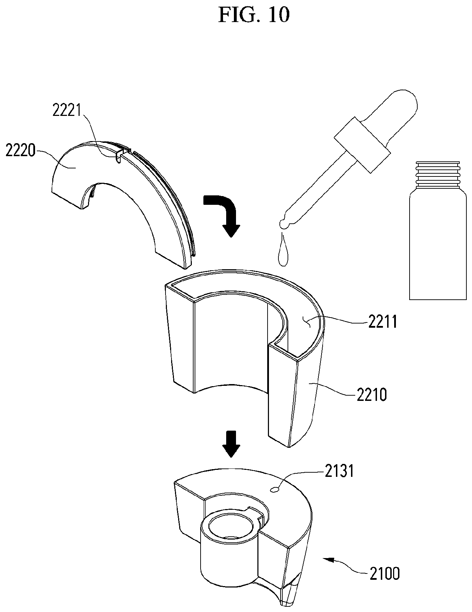

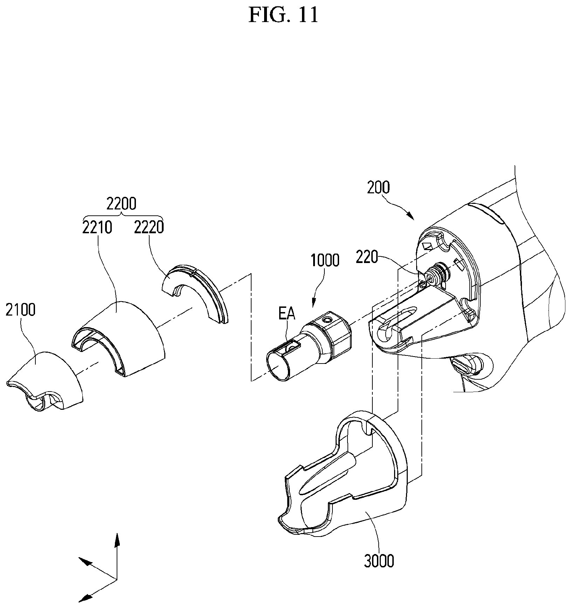

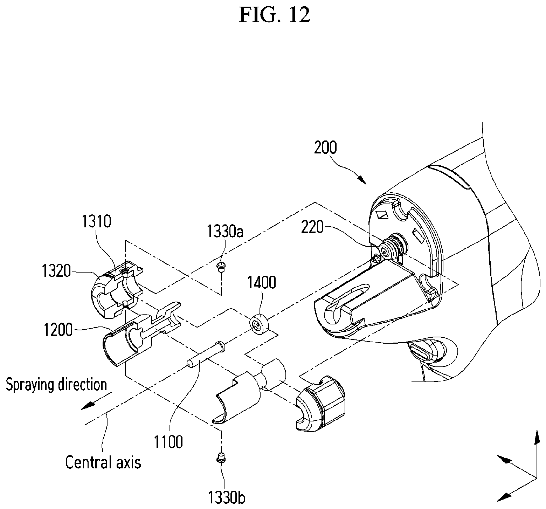

FIG. 9 is a flowchart illustrating a method of using the mixing and spraying system 10 according to the embodiment of the present specification. FIG. 10 is a view illustrating a process in which a composition supply module 2000 is prepared according to the embodiment of the present specification, FIG. 11 is an exploded view of the refrigerant supply device 200 and the mixing module 1000 according to the embodiment of the present specification, and FIG. 12 is a view illustrating a process in which the multi-function module 100 is coupled to the refrigerant supply device 200 according to the embodiment of the present specification.

Referring to FIG. 9, the method of using the mixing and spraying system 10 may a step of coupling the casing 2210 to the adapter 2100 at S110, a step of injecting a composition into the casing 2210 and sealing the casing 2210 at S120, a step of coupling the mixing module 1000 to the refrigerant supply device 200 at S130, a step of coupling the guide unit 3000 to the refrigerant supply device 200 at S140, a step of coupling the composition supply module 2000 to the mixing module 1000 at S150, and a step of mixing and spraying a refrigerant and the composition at S160.

Hereinafter, each step will be described in detail.

First, the casing 2210 may be coupled to the adapter 2100 at S110. For example, referring to FIG. 10, in order to seal the first end of the casing 2210, the casing 2210 may be coupled to the adapter body 2110. Specifically, the adapter body 2110 includes a step or holding part of a shape corresponding to the first end of the casing 2210, and the casing 2210 may be coupled to the adapter body 2110 by a forcible fitting method. In this case, the composition receiving space 2211 of the casing 2210 may be fluidly connected to the first end 2131 of the inlet unit, and the diameter of the first end 2131 of the inlet unit is small enough to minimize leakage of a composition.

Next, a composition may be injected into the casing 2210 and the casing 2210 may be sealed at S120. For example, referring to FIG. 10, the composition may be injected into the casing 2210, the first end of which is sealed by the adapter 2100. A composition may be sourced from a cosmetic product or a medical product. After the composition is injected into the sealed casing 2210, the cap part 2220 is coupled to the casing 2210 so that the casing 2210 can be sealed. Meanwhile, the sealed casing 2210 may be fluidly connected to the outside through the inlet unit 2130 of the adapter 2100 at the first end thereof, and may be fluidly connected to the outside through the outside air inlet unit 2221 of the cap part 2220 at the second end thereof.

The step S110 and the step S120 may be understood as steps to prepare the composition supply module 2000. In other words, the composition supply module 2000 in a state in which a composition is stored may be provided through the step S110 and the step S120.

Next, the mixing module 1000 may be coupled to the refrigerant supply device 200 at S130. The refrigerant supply device 200 may be prepared in a state in which the nozzle 210 is separated from the refrigerant supply device 200 so that refrigerant supply device 200 is coupled to the mixing module 1000.

For example, the combining unit 1300 of the mixing module 1000 may be coupled to the module combining unit 220 of the refrigerant supply device 200 through screw coupling. For example, the combining unit 1300 may include a female screw part or a male screw part, and the module combining unit 220 may include a male screw part or a female screw part corresponding to the combining unit 1300. Here, the mixing module 1000 and the module combining unit 220 may be coupled to each other in a coaxial arrangement. Specifically, referring to FIG. 11, with the central axis of the mixing module 1000 and the central axis of the module combining unit 220 arranged on the same line, the main combining part 1310 of the mixing module 1000 rotates and is screwed to the module combining unit 220, and accordingly, the auxiliary combining part 1320 may press the mixing unit 1200 in a direction opposite to the spraying direction to fix the spraying unit 1100 and the supporting unit 1400 to the module combining unit 220.

For another example, a module combining unit 1300 of the mixing module 1000 may be coupled to the module combining unit 220 of the refrigerant supply device 200 through forcible fitting, slide coupling, or magnetic coupling.

Meanwhile, when the mixing module 1000 is coupled to the refrigerant supply device 200, the supporting unit 1400 may be disposed between the spraying unit 1100 and the module combining unit 1300. Since the supporting unit 1400 is disposed between the spraying unit 1100 and the module combining unit 1300, the spraying unit 1100 and the module combining unit 1300 may be prevented from being damaged by an external force or movement of a refrigerant. The supporting unit 1400 may include a shape corresponding to each of the spraying unit 1100 and the module combining unit 1300. Specifically, when the first end of the supporting unit 1400 contacts the spraying unit 1100, the first end of the supporting unit 1400 may include a first groove for accommodating at least a portion of the spraying unit 1100. Additionally, when the second end of the supporting unit 1400 contacts the module combining unit 1300, the second end of the supporting unit 1400 may include a second groove for accommodating at least a portion of the module combining unit 1300.

After the mixing module 1000 is coupled to the refrigerant supply device 200, the guide unit 3000 may be coupled to the refrigerant supply device 200 at S140. The guide unit 3000 may be coupled to the refrigerant supply device 200 in various ways such as coupling by forcible fitting, screw coupling, magnet coupling, and/or slide coupling, etc. For example, referring to FIG. 12, the main body MB of the refrigerant supply device 200 has a magnetic material, and the guide unit 3000 has a magnetic material corresponding thereto, and thus the guide unit 3000 may be magnetically coupled to the refrigerant supply device 200. According to the embodiment of the present disclosure, the main body MB of the refrigerant supply device 200 may be provided with a magnetic material which is not exposed to the outside, and the guide unit 3000 may be provided with a magnetic material corresponding thereto which is exposed to the outside, but this is not limiting.

In a state where the mixing module 1000 is coupled to the refrigerant supply device 200, the composition supply module 2000 may be coupled to the mixing module 1000 at S150. As described above, the composition supply module 2000 may be mounted on the mounting area EA of the mixing module 1000. For example, referring to FIG. 12, the composition supply module 2000 may be mounted on the mounting area EA of the mixing module 1000 through slide coupling. In this case, a direction in which the composition supply module 2000 is mounted on the mixing module 1000 may be a direction opposite to the spraying direction.

Meanwhile, the direction in which the composition supply module 2000 is mounted on the mixing module 1000 is not limited to the direction opposite to the spraying direction. For example, the composition supply module 2000 may be mounted on the mixing module 1000 in a direction oblique or perpendicular to the spraying direction. In addition, the composition supply module 2000 may be mounted to the mixing module 1000 by screw coupling, coupling by forcible fitting, and/or magnetic coupling in addition to slide coupling.

However, the degree of protrusion of the composition supply module 2000 from the central axis may be smaller when the composition supply module 2000 is mounted in a direction parallel to the spraying direction than when the composition supply module 2000 is mounted in a direction different from the spraying direction. When the mixing space MA of the mixing module 1000 in which a refrigerant and a composition are mixed is required to have a certain size, a distance between an end of the composition supply module 2000 and the central axis is greater when the composition supply module 2000 is coupled in a direction not parallel to the spraying direction than when the composition supply module 2000 is coupled in a direction parallel to the spraying direction. As the composition supply module 2000 protrudes more from the central axis at which the refrigerant and the composition are sprayed, a user's view may be blocked while the mixing and spraying system 10 is used, and accordingly, it is more preferable that the composition supply module 2000 is mounted in a direction parallel to the spraying direction.

In addition, when considering usability or convenience, a slide coupling is more preferable than a screw coupling or coupling by forcible fitting, and a slide coupling is more preferable than magnetic coupling when considering rigidity or stability.

As a result, it is preferable that the composition supply module 2000 be mounted to the mixing module 1000 in a direction parallel to the spraying direction through slide coupling, but the technical spirit of the present specification is not limited thereto.

Finally, a refrigerant and a composition may be mixed and sprayed at S160. After the multi-function module 100 is coupled to the refrigerant supply device 200, the refrigerant supply device 200 may operate similarly as described through FIG. 3. Specifically, the refrigerant supply device 200 receives an input signal according to a user's manipulation, sets the target cooling temperature and the target cooling time period, and controls the flow rate control unit 240 so that a refrigerant stored in the cartridge CTR can be provided to the multi-function module 100. The multi-function module 100 may mix a refrigerant provided from the refrigerant supply device 200 with a composition and spray the mixture. The process of mixing and spraying a refrigerant and a composition in the multi-function module 100 will be described later.

In the step of mixing and spraying a refrigerant and a composition at S160, the temperatures of the refrigerant and the composition may be controlled. For example, the refrigerant supply device 200 measures the temperature of a target to which the refrigerant and the composition are sprayed through the sensor unit 260, and controls the temperature control unit 230 based on the measured temperature so that the temperatures of the refrigerant and the composition can be controlled. Here, the temperatures of the refrigerant and the composition may be adjusted according to preset target cooling temperatures, and the target cooling temperature may be determined according to the purpose of use of the mixing and spraying system 10, a composition used or the type of an active ingredient included in the composition.

When mixing and spraying the refrigerant and the composition, the target cooling temperature may be set within an appropriate range.

The target cooling temperature may be determined between -30°C and 30°C. Alternatively, the target cooling temperature may be determined between -30°C and 25°C, between -30°C and 20°C, between -30°C and 15°C, between -30°C and 10°C, between - 30°C and 5°C, between -30°C and o°C, between -30°C and -5°C, between -30°C and -10°C, between -30°C and -15°C, between -30°C and -20°C, between -30°C and -25°C, between - 25°C and 30°C, between -20°C and 30°C, between -15°C and 30°C, between -10°C and 30°C, between -5°C and 30°C, between o°C and 30°C, between 5°C and 30°C, between 10°C and 30°C, between 15°C and 30°C, between 20°C and 30°C, or between 25°C and 30°C.

Alternatively, the target cooling temperature may be set to be -30°C, -25°C, -20°C, -15°C, -10°C, -5°C, 0°C, 5°C, 10°C, 15°C, 20°C, 25°C, or 30°C.

Meanwhile, the refrigerant supply device 200 may control the temperature of a target to be within a preset temperature range, instead of controlling the temperature of the target to be the preset target cooling temperature.

Here, the temperature range may be a range selected between -30°C and 30°C. For example, the temperature range may be -30°C to 25°C, -30°C to 20°C, -30°C to 15°C, - 30°C to 10°C, -30°C to 5°C, -30°C to 0°C, -30°C to -5°C, -30°C to -10°C, -30°C to -15°C, - 30°C to -20°C, -30°C to -25°C, -25°C to 30°C, -20°C to 30°C, -15°C to 30°C, -10°C to 30°C, -5°C to 30°C, 0°C to 30°C, 5°C to 30°C, 10°C to 30°C, 15°C to 30°C, 20°C to 30°C, or 25°C to 30°C.

Alternatively, the temperature range may be -5°C to 5°C, 0°C to 8°C, or 2°C to 8°C.

Meanwhile, the above-described steps S110, S120, S130, S140, S150, and S160 do not necessarily have to be performed sequentially and may be performed in a different order as follows.

The step of injecting the composition into the casing 2210 and sealing the casing 2210 at S120 may be performed in parallel with the step of coupling the casing 2210 to the adapter 2100 at S110. For example, in a state in which the second end of the casing 2210 is sealed by the cap part 2220, the composition is injected into the casing 2210, and after the injection of the composition, the adapter 2100 is coupled to the first end of the casing 2210, and thus the composition supply module 2000 may be prepared.

Alternatively, after the guide unit 3000 is coupled to the refrigerant supply device 200, the mixing module 1000 may be coupled to the refrigerant supply device 200. Alternatively, after the composition supply module 2000 is mounted to the mixing module 1000, the mixing module 1000 may be coupled to the refrigerant supply device 200.

### [A design condition of the mixing and spraying system]

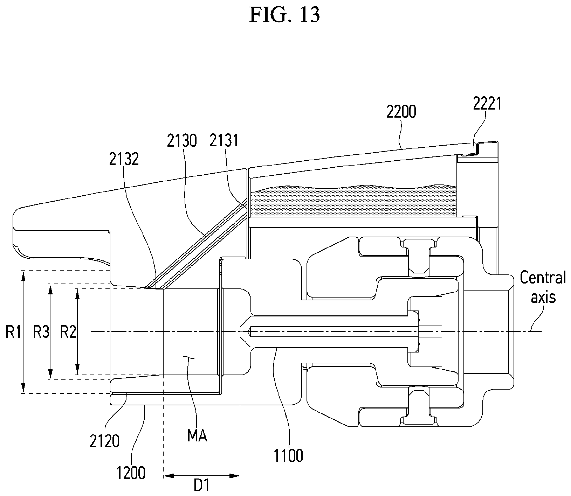

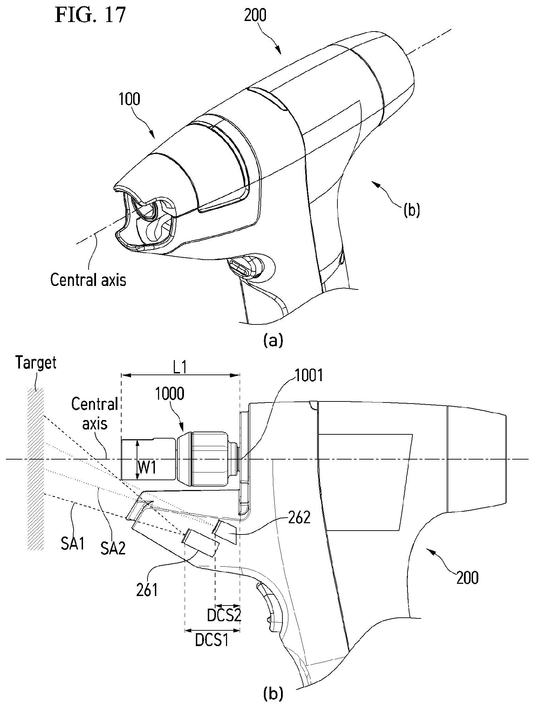

In order for the mixing and spraying system 10 to operate as described above, components of the system is required to be designed to satisfy specific conditions. Hereinafter, a process in which the mixing and spraying system 10 operates and a design condition which the components of the mixing and spraying system 10 is required to satisfy will be described with reference to FIGS. 13 to 17. In FIGS. 13 to 17, a state in which the multi-function module 100 and the refrigerant supply device 200 are coupled to each other is illustrated, and for convenience of description, some components are omitted. In FIGS. 13 to 16, the refrigerant supply device 200 and the guide unit 3000 are omitted, and in FIG. 17(b), the composition supply module 2000 and the guide unit 3000 are omitted.

FIG. 13 is a view illustrating the sections of some components of the mixing and spraying system 10 according to the embodiment of the present specification. FIG. 13 illustrates the composition supply module 2000 and the mixing module 1000 in a state in which a refrigerant and the composition 2 are not sprayed.

The mixing unit 1200 of the mixing module 1000 and the adapter combining unit 2120 of the adapter 2100 may each have preset inner diameters. Referring to FIG. 13, the mixing unit 1200 may have a first inner diameter R1. The adapter combining unit 2120 may be divided into a part having a second inner diameter R2 and a part having an inner diameter gradually increasing from the second inner diameter R2 to a third inner diameter R3.

The mixing module 1000 may provide the mixing space MA. The mixing space MA may be understood to include a space in which a composition and a refrigerant are mixed according to the operation of the mixing and spraying system 10. As described below, the mixing space MA may be an inner space of the mixing unit 1200 or a specific part of the inner space of the mixing unit 1200 and may have a shape or size determined by the mixing unit 1200 and/or the adapter 2100.

The mixing space MA may have a preset width in a direction perpendicular to the central axis of the mixing module 1000. The width of the mixing space MA may correspond to the inner diameter of the mixing unit 1200 of the mixing module 1000 or the inner diameter of the adapter combining unit 2120. For example, as illustrated in FIG. 13, in a case in which the adapter combining unit 2120 is slidably coupled to the mixing module 1000, a portion of the adapter combining unit 2120 has a shape corresponding to the mixing unit 1200, and the portion of the adapter combining unit 2120 is inserted into the mixing unit 1200, the width of the mixing space MA may be determined between the second inner diameter R2 and the third inner diameter R3 of the adapter combining unit 2120. For another example, in a case in which the adapter combining unit 2120 is slidably coupled to the mixing module 1000 and the portion of the adapter combining unit 2120 is not inserted into the mixing unit 1200, the width of the mixing space MA may be determined to be the first inner diameter R1 of the mixing unit 1200. In other words, in a case in which at least a portion of the adapter combining unit 2120 is inserted into the mixing unit 1200, the width of the mixing space MA may be determined by at least the shape or inner diameter of the adapter combining unit 2120, and in a case in which the adapter combining unit 2120 does not affect the inside of the mixing unit 1200, the width of the mixing space MA may be determined by the inner diameter of the mixing unit 1200.

The mixing space MA may be fluidly combined with the composition container 2200. For example, referring to FIG. 13, the mixing space MA may be connected to the casing 2210 of the composition container 2200 through the inlet unit 2130. Specifically, the first end 2131 of the inlet unit may be connected to the casing 2210, and the second end 2132 of the inlet unit may be connected to the mixing space MA.

Here, the spraying unit 1100 and a part of the mixing space MA into which the composition 2 is introduced may have a certain distance therebetween. For example, referring to FIG. 13, based on the central axis, the second end 2132 of the inlet unit may be spaced apart from the end of the spraying unit 1100 by a first distance D1. The first distance D1 is related to the design condition mentioned above, which will be described later.

When a refrigerant is not sprayed, the composition 2 may not be introduced into the mixing space MA. Specifically, the composition 2 stored in the composition container 2200 may not be introduced into the inlet unit 2130 when there is no change in external pressure. In this case, the diameter of the first end 2131 of the inlet unit and/or the diameter of the second end 2132 of the inlet unit may be small enough so that the composition is not introduced thereinto.

The diameter of the inlet unit 2130 may be determined according to the target flow rate of the composition. For example, the flow rate of the composition may be proportional to the four squares of the diameter of the inlet unit 2130, may be inversely proportional to the length of the inlet unit 2130, and may be inversely proportional to the viscosity of the composition, and the diameter of the inlet unit 2130 may be set so that the flow rate of the composition is a preset target flow rate. Specifically, the composition may have at least viscosity of water, and in this case, the diameter of the inlet unit 2130 may be selected between 0.4mm and 1.5mm. The diameter of the inlet unit 2130 may preferably be 0.6mm.

Before describing a process in which the composition 2 is introduced into the mixing space MA, the spraying shape of a refrigerant by the spraying unit 1100 will first be described.

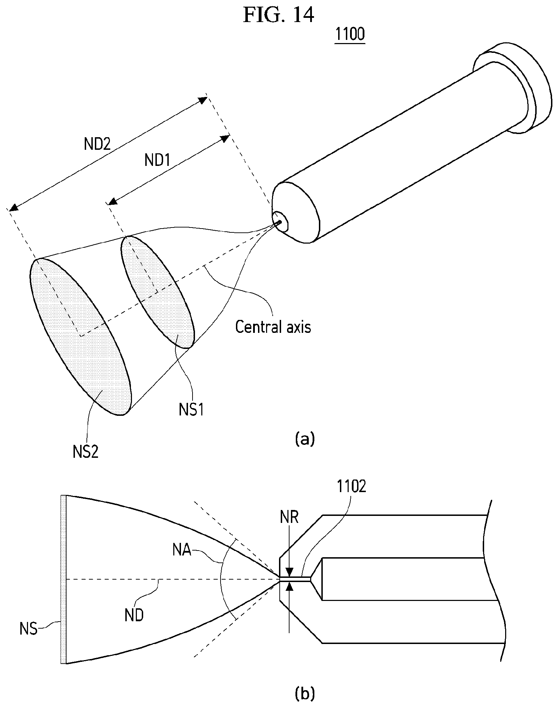

FIG. 14 is a view illustrating the spraying unit 1100 and a spraying shape of a spraying refrigerant according to the embodiment of the present specification.

A refrigerant sprayed from the spraying unit 1100 may have a spraying shape. Referring to FIG. 14(b), the spraying shape of the refrigerant may be formed according to a spraying angle NA and a spraying distance ND.

The spraying angle NA may refer to an angle at which a refrigerant spreads at a moment at which the refrigerant is sprayed from the spraying unit 1100. The spraying angle NA may be determined according to the diameter of the outlet hole 1102 of the spraying unit 1100, an exit angle (or slope) of the outlet hole 1102, and the pressure of a refrigerant, and the like. For example, the spraying angle NA may increase as the exit angle of the outlet hole 1102 increases and as the pressure of a refrigerant increases.

In addition, the exit angle of the spraying unit 1100 may be an angle between 0° and 60°. For example, the exit angle of the spraying unit 1100 may be 0°.

Furthermore, a pressure in the cartridge CTR corresponding to the pressure of a refrigerant may be determined between 35 and 100 bar.

In this case, the spraying angle NA may be determined between 20° and 70°.

The spraying distance ND may mean a distance from the end of the spraying unit 1100.

The spraying shape may be formed over the spraying distance ND along a parabola formed by the spraying angle NA.

The spraying shape may have a spraying area NS according to the spraying distance ND. The spraying area NS may mean an area corresponding to a plane perpendicular to the central axis of the spraying unit 1100 in the spraying shape. In this case, the central axis of the spraying unit 1100 may be understood as the same as the central axis of the mixing module 1000 or the multi-function module 100. Referring to FIG. 14(a), the spraying shape may have the first spraying area NS1 at a first spraying distance ND1 and a second spraying area NS2 at a second spraying distance ND2.

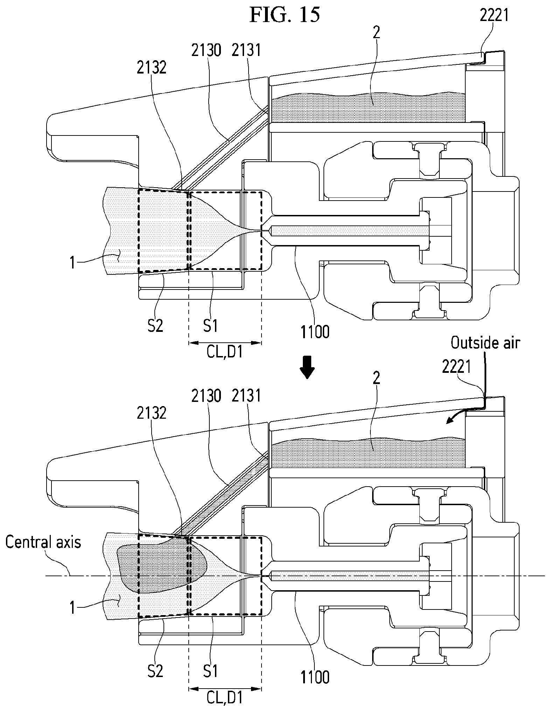

FIG. 15 is a view illustrating a process in which a refrigerant 1 and the composition 2 are mixed with each other according to the embodiment of the present specification.

The refrigerant 1 may be sprayed from the spraying unit 1100. The refrigerant 1 sprayed from the spraying unit 1100 may have a unique spraying shape. Here, the spraying shape may be divided into at least a first section S1 and a second section S2 in consideration of the mixing space MA. For example, referring to FIG. 15, the spraying shape may be divided into the first section S1 having an aspect that the refrigerant 1 spreads in the width direction of the mixing space MA (or in a direction away from the central axis), and the second section S2 having an aspect corresponding to the mixing space MA.

The first section S1 is near the outlet hole 1102 and may be understood as a section in which the refrigerant 1 starts to occupy the mixing space MA. In other words, in the first section S1, the mixing space MA may not be saturated by the refrigerant 1.

At any point in the first section S1, the area of the spraying area NS may not correspond to the width of the mixing space MA. Alternatively, at any point in the first section S1, the area of the spraying area NS may not correspond to a cross-sectional area perpendicular to the central axis of the mixing space MA. Alternatively, at any point in the first section S1, the width of the spraying area NS may not correspond to the width of the mixing space MA.

The second section S2 may be understood as a section after the first section S1 in a spraying direction in the mixing space MA. In the second section S2, the mixing space MA may be saturated by the refrigerant 1. In other words, in the second section S2, the mixing space MA may be occupied by the refrigerant 1.

At any point in the second section S2, the area of the spraying area NS may correspond to the width of the mixing space MA. Alternatively, at any point in the second section S2, the area of the spraying area NS may correspond to the cross-sectional area perpendicular to the central axis of the mixing space MA. Alternatively, at any point in the second section S2, the width of the spraying area NS may correspond to the width of the mixing space MA.

The first section may have a critical length CL. The critical length CL may be determined based on at least the diameter of the outlet hole 1102, the spraying angle NA of the outlet hole 1102, the exit angle of the outlet hole 1102, or the width of the mixing space MA. For example, when the diameter of the outlet hole 1102 is 0.15mm, the exit angle of the outlet hole 1102 is 0°, and the width of the mixing space MA is 8mm, the critical length CL may be 9.5mm.

The critical length CL does not always have a specific value, and, as described above, may have a different value according to specifications of the components of the mixing and spraying system 10. The critical length CL may increase as the diameter of the outlet hole 1102 increases. The critical length CL may decrease as the spraying angle NA increases. The critical length CL may decrease as the exit angle of the outlet hole 1102 increases. The critical length CL may increase as the width of the mixing space MA increases.

As a refrigerant 1 is sprayed, negative pressure may be formed on at least a part of the inner diameter surface of the mixing space. As the refrigerant 1 having a certain speed moves near the inner diameter surface of the mixing space MA, pressure lower than atmospheric pressure may be formed according to Bernoulli's principle. For example, referring to FIG. 15, the refrigerant 1 may be sprayed at a certain speed along the inner diameter surface of the mixing space MA in the second section S2, and negative pressure may be formed accordingly.

Here, as described above, the inlet unit 2130 may be fluidly connected to the mixing space MA, and the second end 2132 of the inlet unit may be located on the inner diameter surface of the mixing space MA. For example, as illustrated in FIG. 15, the second end 2132 of the inlet unit may be located in a portion corresponding to the second section S2 of the inner diameter surface of the mixing space MA. In this case, the first distance D1 between the end of the spraying unit 1100 and the second end 2132 of the inlet unit may be equal to or greater than the critical length CL.

When the refrigerant 1 is sprayed, negative pressure may be formed in the second end 2132 of the inlet unit. Referring to FIG. 15, when negative pressure is formed in the second end 2132 of the inlet unit, pressure inside the composition container 2200 is greater than pressure in the second end 2132 of the inlet unit, so outside air may be introduced through the outside air inlet unit 2221. As outside air is introduced through the outside air inlet unit 2221, the composition 2 may move to the mixing space MA through the inlet unit 2130 and may be mixed with the refrigerant 1.

Meanwhile, according to the position of the second end 2132 of the inlet unit in the mixing space MA, the composition 2 may not be introduced into the mixing space MA even when the refrigerant 1 is sprayed.

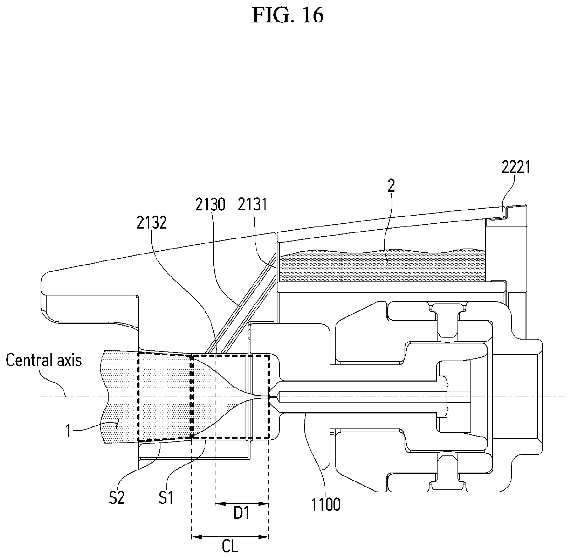

FIG. 16 is a view illustrating a case in which the refrigerant 1 and the composition 2 are not mixed with each other according to the embodiment of the present specification. Referring to FIG. 16, the second end 2132 of the inlet unit may be formed on the inner diameter surface of the mixing space MA corresponding to the first section S1. Alternatively, the first distance D1 between the end of the spraying unit 1100 and the second end 2132 of the inlet unit may be shorter than the critical length CL. In this case, even when the refrigerant 1 is sprayed from the spraying unit 1100, negative pressure is not formed at the second end 2132 of the inlet unit, so the composition 2 may not flow into the mixing space MA through the inlet unit 2130. In other words, the refrigerant 1 does not move near the inner diameter surface of the mixing space MA corresponding to the first section S1, and negative pressure is not formed (or negligible negative pressure is formed) at the second end of the inlet unit, and thus there is no pressure difference between the second end 2132 of the inlet unit and the inside of the composition container 2200 (or there isn't enough pressure difference to move the composition 2), so the composition 2 may not move to the mixing space MA (or the composition 2 may move very slightly).

As described above, in order for the refrigerant 1 and the composition 2 to be mixed in the mixing space MA, the position of the second end 2132 of the inlet unit in the mixing space MA may be very important. The second end 2132 of the inlet unit is required to be located in a part corresponding to the second section S2 of the mixing space MA so that negative pressure is formed at the second end 2132 of the inlet unit according to the spraying of the refrigerant 1. Alternatively, relative to the central axis, the first distance D1 between the end of the spraying unit 1100 and the second end 2132 of the inlet unit may be greater than or equal to the critical length CL of the first section S1.

For example, when the diameter of the outlet hole 1102 is 0.15mm, the exit angle of the outlet hole 1102 is 0°, and the width of the mixing space MA is 8mm, the critical length CL may be 9.5mm and the first distance D1 may be 9.5mm or more.

Although the second end 2132 of the inlet unit has been described to be located on the inner diameter surface of the mixing space MA, the second end 2132 of the inlet unit may be designed to protrude from the inner diameter surface of the mixing space MA toward the central axis. In this case, when the first distance D1 between the end of the spraying unit 1100 and the second end 2132 of the inlet unit is a distance to form negative pressure in the second end 2132 of the inlet unit according to the spraying of the refrigerant 1, the first distance D1 is sufficient, and needs not necessarily be greater than or equal to the critical length CL. However, in this case, the second end 2132 of the inlet unit is cooled, so the composition 2 may not be introduced into the mixing space MA, or only a slight amount of the composition 2 may be introduced thereto. Accordingly, preferably, the second end 2132 of the inlet unit is required to be located on the inner diameter surface of the mixing space MA corresponding to the second section S2 in the spraying shape of the refrigerant 1.

Hereinafter, a design condition regarding a positional relationship between the mixing module 1000 of the multi-function module 100 and the sensor unit 260 of the refrigerant supply device 200 in the components of the mixing and spraying system 10 will be described with reference to FIG. 17.

FIG. 17 is a view illustrating a relation between the position of the mixing module 1000 of the multi-function module 100 and the position of the sensor unit 260 of the refrigerant supply device 200 according to the embodiment of the present specification.

First, as described above, the refrigerant supply device 200 may include the sensor unit 260 to measure the temperature of a target, and the sensor unit 260 may include at least one sensor. In addition, the sensor unit 260 may have a sensing area according to an angle of view (or a wide angle). For example, referring to FIG. 17, the sensor unit 260 may include a first sensor 261 and a second sensor 262. The first sensor 261 may have a first sensing area SA1 and the second sensor 262 may have a second sensing area SA2. The first sensor 261 and the second sensor 262 are disposed to have preset angles, so that the first sensing area SA1 and the second sensing area SA2 may have the same center at a specific distance from the refrigerant supply device 200. In other words, when a target is located at a specific distance from the refrigerant supply device 200, the first sensor 261 and the second sensor 262 may be arranged to measure the temperature of the same part of the target.

Here, the range of the first sensing area SA1 may be determined according to the angle of view (or a wide angle) of the first sensor 261, and the range of the second sensing area SA2 may be determined according to the angle of view (or a wide angle) of the second sensor 262.

Furthermore, the first sensing area SA1 and the second sensing area SA2 may be determined according to distances by which the first and second sensor 261 and 262 respectively are away from the first end 1001 of the mixing module. Specifically, the first sensing area SA1 may be determined by an end of the first sensor 261 spaced apart by a first separation distance DCS1 from the first end 1001 of the mixing module, slope of the first sensor 261 with respect to the central axis, and the angle of view of the first sensor 261, and the second sensing area SA2 may be determined by an end of the second sensor 262 spaced apart by a second separation distance DCS2 from the first end 1001 of the mixing module, slope of the second sensor 262 with respect to the central axis, and the angle of view of the second sensor 262.

Since the refrigerant supply device 200 includes the first sensor 261 and the second sensor 262, more accurate temperature measurement is possible and precise temperature control is possible. However, the refrigerant supply device 200 may use one sensor.

The mixing module 1000 may have a certain length, and the mixing unit 1200 of the mixing module 1000 may have a certain width. For example, referring to FIG. 17, the mixing module 1000 may have a first length L1, and the mixing unit 1200 may have a first width W1. The first length L1 of the mixing module 1000 may be determined according to the length of the mixing unit 1200 and the length of the combining unit 1300. The first width W1 of the mixing unit 1200 may correspond to the outer diameter of the mixing unit 1200.

The mixing module 1000 may be designed not to interfere with the sensing area of the sensor unit 260.

For example, as illustrated in FIG. 17, the first length L1 of the mixing module 1000 and the first width W1 of the mixing unit 1200 may be determined within a range in which the mixing module 1000 does not interfere with the first sensing area SA1 and the second sensing area SA2 of the first sensor 261. Specifically, the first length L1 of the mixing module 1000 and the first width W1 of the mixing unit 1200 may be determined based on the first separation distance DCS1, the slope of the first sensor 261 to the central axis, the angle of view of the first sensor 261, the second separation distance DCS2, the slope of the second sensor 262 to the central axis, and the angle of view of the second sensor 262.

For another example, the first length L1 of the mixing module 1000 and the first width W1 of the mixing unit 1200 may be determined within a range in which a sensing area of any one of a plurality of sensors included in the sensor unit 260 is not covered. Specifically, based on the first separation distance DCS1, the slope of the first sensor 261 to the central axis, and the angle of view of the first sensor 261, the first length L1 of the mixing module 1000 and the first width W1 of the mixing unit 1200 may be determined. Alternatively, based on the second separation distance DCS2, the slope of the second sensor 262 to the central axis, and the angle of view of the second sensor 262, the first length L1 of the mixing module 1000 and the first width W1 of the mixing unit 1200 may be determined.

In the above, a case in which the sensor unit 260 includes two sensors has been described, but the technical spirit of the present specification is not limited thereto, and even when the sensor unit 260 includes one sensor or at least three sensors, the first length L1 of the mixing module 1000 and the first width W1 of the mixing unit 1200 may be respectively determined as values in which the mixing module 1000 does not interfere with the sensing area of the sensor unit 260.

In addition, in the above, the design condition of the length of the mixing module 1000 and the width of the mixing unit 1200 has been described, but the technical spirit of the present specification is not limited thereto, and even when the composition supply module 2000 or the guide unit 3000 is coupled to the mixing module 1000 and affects the sensing area of the sensor unit 260, a numerical value of a specific part of the composition supply module 2000 or the guide unit 3000 may be appropriately adjusted so that the sensing area of the sensor unit 260 is not affected.

### [Another embodiment of the mixing and spraying system]

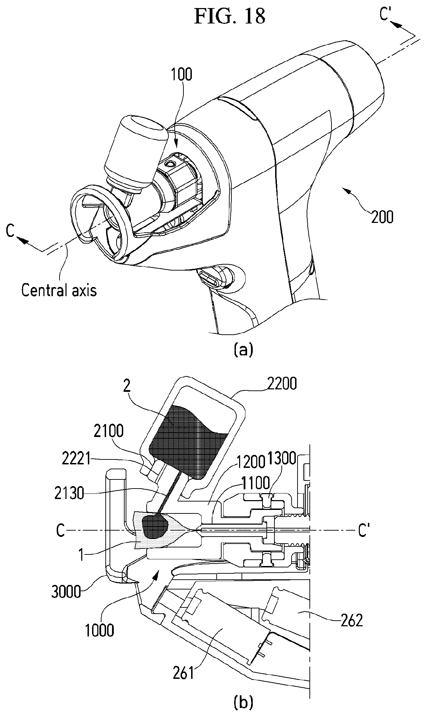

Hereinafter, the another embodiment of the mixing and spraying system 10 will be described with reference to FIGS. 18 to 20.

FIG. 18 is a view illustrating a case in which the outside air inlet unit 2221 is formed in the adapter 2100 in the mixing and spraying system 10 according to the embodiment of the present specification.

As described above, in order for the composition 2 to move to the mixing space MA through the inlet unit 2130, outside air is required to flow into the composition container 2200, and the outside air inlet unit 2221 may provide a flow path for outside air to move to the composition container 2200.

The outside air inlet unit 2221 may be formed in the adapter 2100. For example, referring to FIG. 18, the adapter 2100 may include the outside air inlet unit 2221 which fluidly connects the composition container 2200 to the outside. As described above, when negative pressure is formed in the second end 2132 of the inlet unit according to the injection of the refrigerant 1, the composition 2 may move to the mixing space MA since the first end of the outside air inlet unit 2221 corresponds to atmospheric pressure.

Meanwhile, as illustrated in FIG. 18, the mixing module 1000 and the adapter 2100 may be configured as an integral type, and as described above, the adapter 2100 may be mounted to the mixing module 1000.

In addition, as illustrated in FIG. 18, at least a portion of the adapter 2100 may be combined with the composition container 2200 to seal the composition container 2200. In this case, the composition container 2200 includes the casing 2210 in which the composition 2 is stored and the cover for sealing the casing, and in operating the mixing and spraying system 10, after the cover of the composition container 2200 is removed, the casing 2210 of the composition container 2200 may be combined with the adapter 2100.

Alternatively, unlike the illustration of FIG. 18, the mixing module 1000 may include the inlet unit 2130 and the outside air inlet unit 2221, and the composition container 2200 may be coupled to the mixing module 1000 through the inlet unit 2130 and the outside air inlet unit 2221. For example, the first end of the inlet unit 2130 and the first end of the outside air inlet unit 2221 are respectively provided with piercing members such as a needle, and as the cover of the composition container 2200 is perforated by the inlet unit 2130 and the outside air inlet unit 2221, the composition container 2200 may be coupled to the mixing module 1000.

In this case, the mixing and spraying system 10 may include a support member for supporting the composition container 2200. For example, the support member extends from the mixing module 1000 or the mixing unit 1200, and may support at least a portion of the composition container 2200 when the inlet unit 2130 and the outside air inlet unit 2221 are connected with the composition container 2200.

Here, the composition container 2200 may be a commercially available cosmetic product or medical product such as an ampoule, cosmetic product, or vial.

Even in the case of the mixing and spraying system 10 described through FIG. 18, of course, the design condition of the mixing and spraying system 10 described above is required to be satisfied. For example, a distance between the end of the spraying unit 1100 of the mixing module 1000 and the second end 2132 of the inlet unit may be determined to be greater than or equal to the critical length CL for forming negative pressure in the second end 2132 of the inlet unit according to the spraying shape of the refrigerant 1. For another example, the length and width of the mixing unit 1200 may be determined within a range in which the sensing area of the sensor unit 260 is not blocked.

Meanwhile, in the above, the case in which the composition 2 is introduced into the mixing space MA by the formation of negative pressure and the outside air has been described, but the composition 2 may be introduced into the mixing space MA by another structure in which pressure difference is formed according to the spraying of the refrigerant 1 as described below.

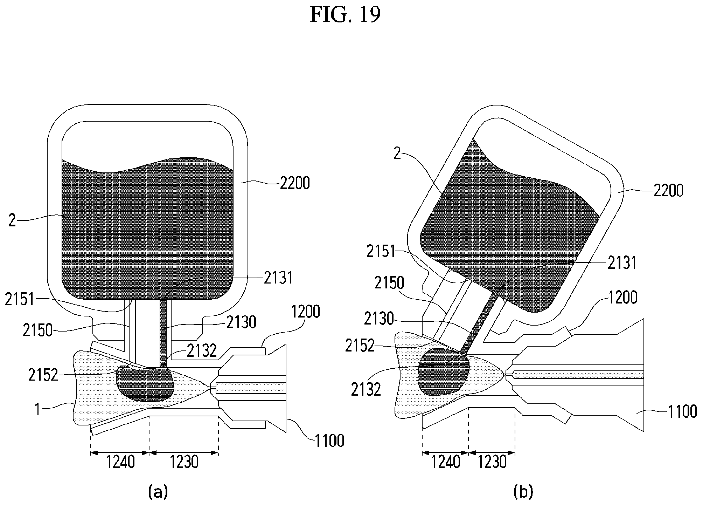

FIG. 19 is a view illustrating the section of the mixing module 1000 according to the embodiment of the present specification.

The mixing module 1000 may include the inlet unit 2130 and an auxiliary flow path 2150 for moving the composition 2. Referring to FIG. 19, the mixing module 1000 may include the inlet unit 2130 through which the composition 2 moves and the auxiliary flow path 2150 for generating pressure difference.

The first end 2151 of the auxiliary flow path may be fluidly connected to the composition container 2200, and the second end 2152 of the auxiliary flow path may be fluidly connected to the mixing space MA of the mixing unit 1200.

The inlet unit 2130 and the auxiliary flow path 2150 are both connected to the mixing space MA, but the width of a part of the mixing space MA to which the inlet unit 2130 is connected and the width of a part of the mixing space MA to which the auxiliary flow path 2150 is connected may be different from each other. For example, among cross sections of the mixing space MA perpendicular to the central axis, an area of a cross section corresponding to the second end 2132 of the inlet unit may be smaller than an area of a cross section corresponding to the second end 2152 of the auxiliary flow path.

In other words, the mixing unit 1200 providing the mixing space MA may include a first part 1230 in which the inlet unit 2130 is located and a second part 1240 in which the auxiliary flow path 2150 is located, wherein the inner diameter of the first part 1230 may be smaller than the inner diameter of the second part 1240. Specifically, referring to FIG. 19(a), the mixing unit 1200 has a shape in which an inner diameter thereof is constant and gradually increases as the distance of the mixing unit 1200 from the spraying unit 1100 increases, and the first part 1230 may be at least a portion of a part having the constant inner diameter, and the second part 1240 may be at least a portion of a part having the inner diameter increasing gradually.

Since the widths of the parts of the mixing space MA connected to the inlet unit 2130 and the auxiliary flow path 2150 are different from each other, pressure difference between the inlet unit 2130 and the auxiliary flow path 2150 may be caused by the spraying of the refrigerant 1. For example, since the flow rate of the refrigerant 1 sprayed from the spraying unit 1100 decreases as the width of the mixing space MA increases, the flow rate of the refrigerant 1 at the second end 2132 of the inlet unit is faster than the flow rate of the refrigerant 1 at the second end 2152 of the auxiliary flow path, and according to Bernoulli's equation, the internal pressure of the inlet unit 2130 is lower than the internal pressure of the auxiliary flow path 2150, and accordingly, the composition 2 may move through the inlet unit 2130 to the mixing space MA.

Meanwhile, both the inlet unit 2130 and the auxiliary flow path 2150 may be formed in the second part 1240 of the mixing unit 1200 in which the inner diameter gradually increases. For example, referring to FIG. 19(b), the second end 2132 of the inlet unit and the second end 2152 of the auxiliary flow path are formed in the second part 1240, and the inner diameter of the part corresponding to the second end 2152 of the auxiliary flow path may be smaller than the inner diameter of the part corresponding to the second end 2132 of the inlet unit.

Of course, even in the case of the mixing and spraying system 10 described through FIG. 19, the design condition of the mixing and spraying system 10 described above is required to be satisfied. For example, the first distance D1 between the end of the spraying unit 1100 of the mixing module 1000 and the second end 2132 of the inlet unit may be determined to be greater than or equal to the critical length CL for forming negative pressure in the second end 2132 of the inlet unit according to the spraying shape of the refrigerant 1. For another example, the length and width of the mixing unit 1200 may be determined within a range in which the sensing area of the sensor unit 260 is not blocked.

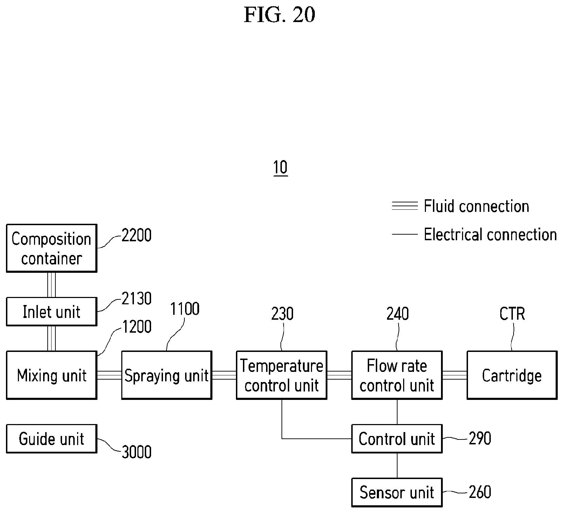

Hereinafter, a case in which the multi-function module 100 and the refrigerant supply device 200 are integrally formed will be described with reference to FIG. 20.

FIG. 20 is a view illustrating a case in which the multi-function module 100 and the refrigerant supply device 200 are integrally configured according to the embodiment of the present specification. Referring to FIG. 20, the mixing and spraying system 10 may include the cartridge CTR, the flow rate control unit 240, the temperature control unit 230, the spraying unit 1100, the mixing unit 1200, the inlet unit 2130, the composition container 2200, the control unit 290, the sensor unit 260, and the guide unit 3000. Since the function of each component has been described above, the function will be omitted unless there are special circumstances.

The cartridge CTR, the flow rate control unit 240, the temperature control unit 230, the spraying unit 1100, the mixing unit 1200, the inlet unit 2130, and the composition container 2200 may be fluidly connected to each other. For example, according to the operation of the mixing and spraying system 10, a refrigerant 1 stored in the cartridge CTR may be moved to the temperature control unit 230 by the flow rate control unit 240, may receive heat from the temperature control unit 230, and may be sprayed to the mixing unit 1200 by the spraying unit 1100. In addition, when the refrigerant 1 is sprayed, the composition 2 stored in the composition container 2200 may be introduced through the inlet unit 2130 into the mixing unit 1200. In this case, when introducing the composition 2 into the mixing unit 1200, the method of using the outside air inlet unit 2221 or the method of using the auxiliary flow path 2150 described above may be selected.

The control unit 290 is electrically connected to the flow rate control unit 240 and the temperature control unit 230 so that the control unit 290 can control the flow rate or temperature of the refrigerant 1. The sensor unit 260 is electrically connected to the control unit 290 so that the sensor unit 260 can provide information necessary for the control unit 290 to control the temperature control unit 230.

Here, the mixing and spraying system 10 may include a housing providing a space for disposing the flow rate control unit 240, the temperature control unit 230, the spraying unit 1100, the mixing unit 1200, the inlet unit 2130, the control unit 290, and the sensor unit 260.

The cartridge CTR may be attached to and detached from the housing, and the housing may include an accommodating space and an accommodating member for accommodating the cartridge CTR. The cartridge CTR may be directly coupled to the flow rate control unit 240 or may be indirectly connected thereto through a conduit.

The composition container 2200 may be attached to and detached from the housing, and may be directly coupled to the inlet unit 2130 or may be indirectly connected thereto through a conduit. The composition container 2200 may be a separate cosmetic or medical product. Alternatively, the composition container 2200 may extend from the housing, and a user may move the composition 2 stored in a cosmetic or medical product to the composition container 2200.

Meanwhile, the temperature control unit 230 may be omitted. In this case, the spraying unit 1100 may be directly coupled to the flow rate control unit 240 or may be indirectly connected thereto through a conduit, etc. Alternatively, the temperature control unit 230 may be disposed in the mixing unit 1200 to provide thermal energy to the mixing space MA.

The guide unit 3000 may be attached to and detached from the housing. Alternatively, the guide unit 3000 may be configured as a part that extends from the housing and maintains a distance from a target. Alternatively, the guide unit 3000 may be omitted.

As described through FIG. 20, even when the multi-function module 100 and the refrigerant supply device 200 are integrally formed, the design condition described above may be satisfied. For example, in the mixing unit 1200, the first distance D1 between the second end 2132 of the inlet unit and the end of the spraying unit 1100 may be greater than or equal to a critical distance CL for forming negative pressure in the second end 2132 of the inlet unit. For another example, the length and width of the mixing unit 1200 may be set within a range in which the sensing area of the sensor unit 260 is not blocked.

The features, structures, effects, etc. described in the embodiments above are included in at least one embodiment of the present specification, and are not necessarily limited to only one embodiment. Furthermore, the embodiments having features, structures, and effects, etc. may be combined or modified with respect to other embodiments by those skilled in the art to which the embodiments belong. Therefore, contents related to these combinations and modifications should be construed as being included in the scope of the present specification.

those skilled in the art to which this specification belongs will know that various modifications not exemplified above are possible within a range without departing from the scope of the present embodiments are not deviated. In addition, differences related to these modifications should be construed as being included within the scope of the present specification as defined in the appended claims.

## Claims

1. A multi-function module (100) **characterized in that** the multi-function module (100) is configured to be coupled to a refrigerant supply device (200), mix a composition with a refrigerant supplied from the refrigerant supply device (200) and spray the mixture to an outside, the module (100) comprising:
a mixing module (1000) including:
a mixing unit (1200) providing a mixing space (MA) for the refrigerant and the composition;
a combining unit (1300) comprising a combining means to combine with the refrigerant supply device (200); and
a spraying unit (1100) disposed between the mixing space (MA) and the combining means and configured to spray the refrigerant supplied from the refrigerant supply device (200) into the mixing space (MA); and
a composition supply module (2000) including:
an adapter (2100) including an adapter body (2110) and an adapter combining unit (2120); and
an inlet unit (2130) providing a flow path of the composition to the mixing space;
wherein the inlet unit (2130) is mounted in the adapter combining unit (2120) and the adapter (2100) is fluidically connected to the mixing unit (1200) by the inlet unit (2130);
wherein the spraying unit (1100) comprises an inlet hole (1101) having a first diameter and an outlet hole (1102) having a second diameter smaller than the first diameter,
wherein the refrigerant sprayed through the outlet hole (1102) forms a negative pressure at a second end of the inlet unit (2130) such that the composition moves into the flow path through a first end of the inlet unit (2130) and flows into the mixing space (MA) through the second end of the inlet unit (2130),
wherein the refrigerant sprayed through the spraying unit (1100) has a unique spraying shape formed along a central axis of the spraying unit (1100),
wherein the spraying shape has a spraying area (NS) perpendicular to the central axis of the spraying unit (1100) and having an area determined according to a distance between the spraying area (NS) and the outlet hole (1102),
wherein the spraying area (NS) is separated into a first section and a second section,
wherein in the first section, the area of the spraying area increases as the distance between the spraying area and the outlet hole increases, and in the second section, the area of the spraying area corresponds to a width of the mixing space (MA), and
wherein the second end of the inlet unit (2130) is formed at a position allowing the composition to flow into the second section.

2. The multi-function module of claim 1,
wherein an area of a first spraying area corresponding to a first point at the second section corresponds to a width of the mixing space (MA) at the first point.

3. The multi-function module of claim 1,
wherein a length of the first section is determined as a critical length when the area of the spraying area corresponds to the width of the mixing space (MA).

4. The multi-function module of claim 3,
wherein the critical length is set based on at least the second diameter of the outlet hole (1102), an exit angle of the outlet hole (1102), and a pressure of the refrigerant supplied.

5. The multi-function module of claim 3,
wherein when the second diameter is 0.15 mm, an exit angle is 0°, and a pressure of the refrigerant is 60bar, a distance from the outlet hole (1102) to the second end of the inlet unit is 9.0mm or more.

6. The multi-function module of claim 1,
wherein a container in which the composition is accommodated is mounted to the adapter body (2110).

7. The multi-function module of claim 6,
wherein the mixing unit (1200) comprises a slide part,
wherein the adapter (2100) comprises a slide groove that is slidably coupled to the slide part of the mixing unit (1200).

8. The multi-function module of claim 7,
wherein the adapter combining unit (2120) has the slide groove,
wherein at least a part of the inlet unit (2130) is located inside the adapter combining unit (2120).

9. The multi-function module of claim 8,
wherein the adapter (2100) comprises an auxiliary guide unit (2140),
wherein the auxiliary guide unit (2140) protrudes compared to the mixing unit (1200).

10. The multi-function module of claim 1, further comprising:
a support member extended from the mixing unit (1200) and configured to support a container (2200) accommodating the composition.

11. The multi-function module of claim 1,
wherein the combining means is a male screw or a female screw such that the combining unit (1300) and the refrigerant supply device (200) are coupled to each other through screw coupling.

12. The multi-function module of claim 1, further comprising:
a supporting unit (1400) disposed between the inlet hole (1101) of the spraying unit (1100) and the refrigerant supply device (200),
wherein the supporting unit (1400) comprises a hollow for the refrigerant to move,
wherein a diameter of the hollow is equal to or greater than the first diameter.

## Patentansprüche

1. Multifunktionsmodul (100), **dadurch gekennzeichnet, dass** das Multifunktionsmodul (100) dazu konfiguriert ist, mit einer Kältemittelzufuhrvorrichtung (200) gekoppelt zu werden, eine Zusammensetzung mit einem Kältemittel zu mischen, das von der Kältemittelzufuhrvorrichtung (200) zugeführt wird, und die Mischung nach außen zu sprühen, wobei das Modul (100) Folgendes umfasst:
ein Mischmodul (1000), das Folgendes enthält:
eine Mischeinheit (1200), die einen Mischraum (MA) für das Kältemittel und die Zusammensetzung bereitstellt;
eine Kombinationseinheit (1300), die ein Kombinationsmittel zum Kombinieren mit der Kältemittelzufuhrvorrichtung (200) umfasst; und
eine Sprüheinheit (1100), die zwischen dem Mischraum (MA) und dem Kombinationsmittel angeordnet und dazu konfiguriert ist, das von der Kältemittelzufuhrvorrichtung (200) zugeführte Kältemittel in den Mischraum (MA) zu sprühen; und
ein Zusammensetzungszufuhrmodul (2000), das Folgendes enthält:
einen Adapter (2100), der einen Adapterkörper (2110) und eine Adapterkombinationseinheit (2120) enthält; und
eine Einlasseinheit (2130), die einen Strömungsweg der Zusammensetzung zu dem Mischraum bereitstellt;
wobei die Einlasseinheit (2130) in der Adapterkombinationseinheit (2120) montiert ist und der Adapter (2100) durch die Einlasseinheit (2130) mit der Mischeinheit (1200) in Fluidverbindung steht;
wobei die Sprüheinheit (1100) ein Einlassloch (1101), das einen ersten Durchmesser aufweist, und ein Auslassloch (1102) umfasst, das einen zweiten Durchmesser aufweist, der kleiner als der erste Durchmesser ist,
wobei das durch das Auslassloch (1102) gesprühte Kältemittel einen Unterdruck an einem zweiten Ende der Einlasseinheit (2130) ausbildet, sodass sich die Zusammensetzung durch ein erstes Ende der Einlasseinheit (2130) in den Strömungsweg bewegt und durch das zweite Ende der Einlasseinheit (2130) in den Mischraum (MA) strömt,
wobei das durch die Sprüheinheit (1100) gesprühte Kältemittel eine einzigartige Sprühform aufweist, die entlang einer Mittelachse der Sprüheinheit (1100) ausgebildet ist,
wobei die Sprühform eine Sprühfläche (NS) aufweist, die senkrecht zu der Mittelachse der Sprüheinheit (1100) ist und eine Fläche aufweist, die gemäß einem Abstand zwischen der Sprühfläche (NS) und dem Auslassloch (1102) bestimmt ist,
wobei die Sprühfläche (NS) in einen ersten Abschnitt und einen zweiten Abschnitt getrennt ist,
wobei in dem ersten Abschnitt die Fläche der Sprühfläche mit zunehmendem Abstand zwischen der Sprühfläche und dem Auslassloch zunimmt und in dem zweiten Abschnitt die Fläche der Sprühfläche einer Breite des Mischraums (MA) entspricht, und
wobei das zweite Ende der Einlasseinheit (2130) an einer Position ausgebildet ist, die es der Zusammensetzung ermöglicht, in den zweiten Abschnitt zu strömen.

2. Multifunktionsmodul nach Anspruch 1,
wobei eine Fläche einer ersten Sprühfläche, die einem ersten Punkt an dem zweiten Abschnitt entspricht, einer Breite des Mischraums (MA) an dem ersten Punkt entspricht.

3. Multifunktionsmodul nach Anspruch 1,
wobei eine Länge des ersten Abschnitts als eine kritische Länge bestimmt ist, wenn die Fläche der Sprühfläche der Breite des Mischraums (MA) entspricht.

4. Multifunktionsmodul nach Anspruch 3,
wobei die kritische Länge basierend auf mindestens dem zweiten Durchmesser des Auslasslochs (1102), einem Austrittswinkel des Auslasslochs (1102) und einem Druck des zugeführten Kältemittels eingestellt ist.

5. Multifunktionsmodul nach Anspruch 3,
wobei, wenn der zweite Durchmesser 0,15 mm beträgt, ein Austrittswinkel 0° beträgt und ein Druck des Kältemittels 60 bar beträgt, ein Abstand von dem Auslassloch (1102) zu dem zweiten Ende der Einlasseinheit 9,0 mm oder mehr beträgt.

6. Multifunktionsmodul nach Anspruch 1,
wobei ein Behälter, in dem die Zusammensetzung untergebracht ist, an dem Adapterkörper (2110) montiert ist.

7. Multifunktionsmodul nach Anspruch 6,
wobei die Mischeinheit (1200) ein Schiebeteil umfasst,
wobei der Adapter (2100) eine Schiebenut umfasst, die verschiebbar mit dem Schiebeteil der Mischeinheit (1200) gekoppelt ist.

8. Multifunktionsmodul nach Anspruch 7,
wobei die Adapterkombinationseinheit (2120) die Schiebenut aufweist,
wobei sich mindestens ein Teil der Einlasseinheit (2130) innerhalb der Adapterkombinationseinheit (2120) befindet.

9. Multifunktionsmodul nach Anspruch 8,
wobei der Adapter (2100) eine Hilfsführungseinheit (2140) umfasst,
wobei die Hilfsführungseinheit (2140) im Vergleich zu der Mischeinheit (1200) vorsteht.

10. Multifunktionsmodul nach Anspruch 1, ferner umfassend:
ein Stützelement, das sich von der Mischeinheit (1200) erstreckt und dazu konfiguriert ist, einen Behälter (2200) zu stützen, der die Zusammensetzung unterbringt.

11. Multifunktionsmodul nach Anspruch 1,
wobei das Kombinationsmittel eine Außengewindeschraube oder eine Innengewindeschraube ist, sodass die Kombinationseinheit (1300) und die Kältemittelzufuhrvorrichtung (200) durch eine Schraubkupplung miteinander gekoppelt sind.

12. Multifunktionsmodul nach Anspruch 1, ferner umfassend:
eine Stützeinheit (1400), die zwischen dem Einlassloch (1101) der Sprüheinheit (1100) und der Kältemittelzufuhrvorrichtung (200) angeordnet ist,
wobei die Stützeinheit (1400) einen Hohlraum umfasst, in dem sich das Kältemittel bewegen kann,
wobei ein Durchmesser des Hohlraums gleich oder größer als der erste Durchmesser ist.

## Revendications

1. Module multifonction (100) **caractérisé en ce que** le module multifonction (100) est configuré pour être couplé à un dispositif d'alimentation en fluide réfrigérant (200), mélanger une composition avec un fluide réfrigérant fourni par le dispositif d'alimentation en fluide réfrigérant (200) et pulvériser le mélange à l'extérieur, le module (100) comprenant :
un module de mélange (1000), comprenant :
une unité de mélange (1200) fournissant un espace de mélange (MA) pour le fluide réfrigérant et la composition ;
une unité de combinaison (1300) comprenant un moyen de combinaison à combiner avec le dispositif d'alimentation en fluide réfrigérant (200) ; et
une unité de pulvérisation (1100) disposée entre l'espace de mélange (MA) et les moyens de combinaison et configurée pour pulvériser le fluide réfrigérant fourni par le dispositif d'alimentation en fluide réfrigérant (200) dans l'espace de mélange (MA) ; et
un module d'alimentation en composition (2000), comprenant :
un adaptateur (2100) comprenant un corps (2110) d'adaptateur et une unité de combinaison (2120) d'adaptateur ; et
une unité d'entrée (2130) fournissant un trajet d'écoulement de la composition jusqu'à l'espace de mélange ;
ladite unité d'entrée (2130) étant montée dans l'unité de combinaison (2120) d'adaptateur et ledit adaptateur (2100) étant raccordé fluidiquement à l'unité de mélange (1200) par l'unité d'entrée (2130) ;
ladite unité de pulvérisation (1100) comprenant un trou d'entrée (1101) possédant un premier diamètre et un trou de sortie (1102) possédant un second diamètre inférieur au premier diamètre,
ledit fluide réfrigérant pulvérisé à travers le trou de sortie (1102) formant une pression négative au niveau d'une seconde extrémité de l'unité d'entrée (2130) de sorte que la composition se déplace dans le trajet d'écoulement à travers une première extrémité de l'unité d'entrée (2130) et s'écoule dans l'espace de mélange (MA) à travers la seconde extrémité de l'unité d'entrée (2130),
ledit fluide réfrigérant pulvérisé à travers l'unité de pulvérisation (1100) possédant une forme de pulvérisation unique formée le long d'un axe central de l'unité de pulvérisation (1100),
ladite forme de pulvérisation possédant une zone de pulvérisation (NS) perpendiculaire à l'axe central de l'unité de pulvérisation (1100) et possédant une surface déterminée selon une distance entre la zone de pulvérisation (NS) et le trou de sortie (1102),
ladite zone de pulvérisation (NS) étant séparée en une première section et une seconde section,
dans la première section, ladite surface de la zone de pulvérisation augmentant à mesure que la distance entre la zone de pulvérisation et le trou de sortie augmente, et dans la seconde section, ladite surface de la zone de pulvérisation correspondant à une largeur de l'espace de mélange (MA), et
ladite seconde extrémité de l'unité d'entrée (2130) étant formée au niveau d'une position permettant à la composition de s'écouler dans la seconde section.

2. Module multifonction de la revendication 1,
une surface d'une première zone de pulvérisation qui correspond à un premier point au niveau de la seconde section correspondant à une largeur de l'espace de mélange (MA) au niveau du premier point.

3. Module multifonction de la revendication 1,
une longueur de la première section étant déterminée en tant que longueur critique lorsque la surface de la zone de pulvérisation correspond à la largeur de l'espace de mélange (MA).

4. Module multifonction de la revendication 3,
ladite longueur critique étant définie sur la base d'au moins le second diamètre du trou de sortie (1102), d'un angle de sortie du trou de sortie (1102) et d'une pression du fluide réfrigérant fourni.

5. Module multifonction de la revendication 3,
lorsque le second diamètre est de 0,15 mm, un angle de sortie est de 0°, et une pression du fluide réfrigérant est de 60 bar, une distance allant du trou de sortie (1102) à la seconde extrémité de l'unité d'entrée étant supérieure ou égale à 9,0 mm.

6. Module multifonction de la revendication 1,
un récipient dans lequel la composition est reçue étant monté sur le corps (2110) d'adaptateur.

7. Module multifonction de la revendication 6,
ladite unité de mélange (1200) comprenant une partie de coulissement,
ledit adaptateur (2100) comprenant une rainure de coulissement qui est couplée de manière coulissante à la partie coulissante de l'unité de mélange (1200).

8. Module multifonction de la revendication 7,
ladite unité de combinaison (2120) d'adaptateur comportant la rainure de coulissement,
au moins une partie de l'unité d'entrée (2130) étant située à l'intérieur de l'unité de combinaison (2120) d'adaptateur.

9. Module multifonction de la revendication 8,
ledit adaptateur (2100) comprenant une unité de guidage auxiliaire (2140), l'unité de guidage auxiliaire (2140) faisant saillie par rapport à l'unité de mélange (1200).

10. Module multifonction de la revendication 1, comprenant en outre :
un élément de support s'étendant à partir de l'unité de mélange (1200) et configuré pour supporter un récipient (2200) recevant la composition.

11. Module multifonction de la revendication 1,
ledit moyen de combinaison étant une vis mâle ou une vis femelle de sorte que l'unité de combinaison (1300) et le dispositif d'alimentation en fluide réfrigérant (200) soient couplés l'un à l'autre par l'intermédiaire d'un accouplement à vis.

12. Module multifonction de la revendication 1, comprenant en outre :
une unité de support (1400) disposée entre le trou d'entrée (1101) de l'unité de pulvérisation (1100) et le dispositif d'alimentation en fluide réfrigérant (200),
ladite unité de support (1400) comprenant un creux pour que le réfrigérant se déplace,
un diamètre du creux étant supérieur ou égal au premier diamètre.
